(19) Europäisches Patentamt
European Patent Office
Office européen des brevets

(11) Veröffentlichungsnummer: **0 315 574**
**A2**

# (12) EUROPÄISCHE PATENTANMELDUNG

(21) Anmeldenummer: **88710042.8**

(22) Anmeldetag: **29.10.88**

(51) Int. Cl.4: **C 07 K 5/02**
C 07 K 5/06, A 61 K 37/64

(30) Priorität: **05.11.87 DE 3737498**
**31.05.88 DE 3818436**

(43) Veröffentlichungstag der Anmeldung:
**10.05.89 Patentblatt 89/19**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80 (DE)**

(72) Erfinder: **Kleemann, Heinz-Werner, Dr.**
**Jahnstrasse 6**
**D-6092 Kelsterbach (DE)**

**Urbach, Hans-Jörg, Dr.**
**Le Lavandoustrasse 41**
**D-6242 Kronberg/Taunus (DE)**

**Ruppert, Dieter, Dr.**
**Schreyerstrasse 30**
**D-6242 Kronberg/Taunus (DE)**

**Schölkens, Bernward, Dr.**
**Hölderlinstrasse 62**
**D-6233 Kelkheim (Taunus) (DE)**

Patentansprüche für folgende Vertragsstaaten: ES + GR.

## (54) Renin-Inhibitoren.

(57) Die Erfindung betrifft Verbindungen der Formel I

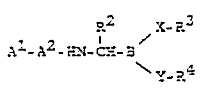

(I)

worin $A^1$ einen der Rest der Formeln 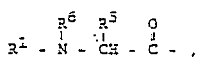 ,

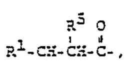 , 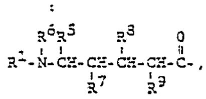 , 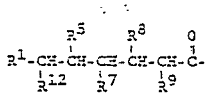 oder

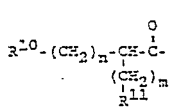 bedeutet,

$A^2$ abwesend ist oder für einen Rest der Formel 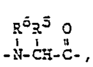 ,

steht, $R^2$, $R^3$ und $R^4$ wie in der Beschreibung definiert sind und X und Y unabhängig voneinander für -O- oder -$NR^{13}$ stehen, sowie deren Salze. Es wird ferner ein Verfahren zur Herstellung der Verbindungen der Formel I, entsprechende pharmazeutische Präparate und deren Verwendung als Heilmittel sowie Zwischenprodukte zur Herstellung dieser Verbindungen beschrieben.

EP 0 315 574 A2

## Renin-Inhibitoren

Die Erfindung betrifft α-Aminoboronsäure-Derivate, die die Wirkung des natürlichen Enzyms Renin hemmen.

Elastase-inhibierende α-Aminoboronsäure-Peptide sind in der US-Patentschrift 4,499,082 beschrieben.

In J. Med. Chem. 30, 1287 (1987) werden die Struktur/Wirkungs-Beziehungen auf dem Gebiet der Renin-Inhibitoren diskutiert. Besondere Beachtung findet die Tatsache, daß potente Inhibitoren auf eine Bindung an die P2′ und P3′-Taschen des Renins nicht verzichten können. Daher sind die bisher bekannten Renin-Inhibitoren mit dem Mangel eines hohen Molekulargewichts und daraus resultierender unzureichender enteraler Bioverfügbarkeit und schneller Gallenausscheidung behaftet. Einzige Ausnahme bilden Aldehyde mit Renin-Inhibitor-Wirkung, die ihrerseits jedoch chemisch labil sind und zur Epimerisierung neigen.

Es wurden erstmals stabile, nicht epimerisierende, potente Renin-Inhibitoren gefunden, die auf eine Bindung an die P′-Taschen des Enzyms verzichten und daher die Grundvoraussetzungen für eine hohe enterale Bioverfügbarkeit erfüllen.

Die Erfindung betrifft Verbindungen der Formel I

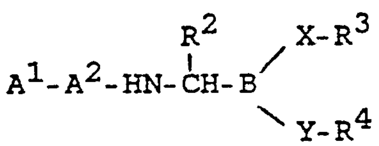

(I)

in welcher

$A^1$ einen Rest der Formeln II, III, IV, V oder VI bedeutet

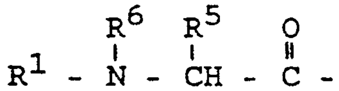

(II)

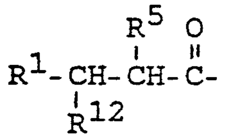

(III)

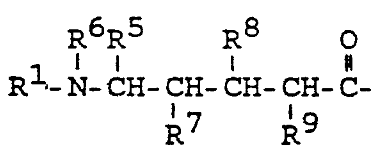

(IV)

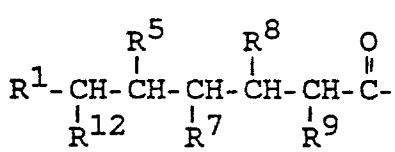

(V)

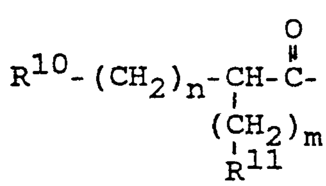

(VI)

worin

$R^1$ $a_1$) Wasserstoff, ($C_1$-$C_{12}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, Carbamoyl, ($C_1$-$C_8$)-Alkanoyloxy, Carboxy, ($C_1$-$C_7$)-Alkoxycarbonyl, F, Cl, Br, I, Amino, Amidino, das gegebenenfalls durch einen, zwei oder drei ($C_1$-$C_8$)-Alkylreste substituiert sein kann, Guanidino, das gegebenenfalls durch einen, zwei, drei oder vier ($C_1$-$C_8$)-Alkylreste substituierte sein kann, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethoxycarbonylamino substituiert ist; mono-, bi- oder tricyclisches ($C_3$-$C_{18}$)-Cycloalkyl, ($C_3$-$C_{18}$)-Cycloalkyl-($C_1$-$C_6$)-alkyl oder ($C_6$-$C_{14}$)-Aryl, wobei der Cycloalkylteil gegebenenfalls durch ($C_1$-$C_6$)-Alkyl substituiert ist und Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogenen substituiertes Anilino und Trifluormethyl substituiert ist; ($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5- oder 6-gliedrigen monocyclischen oder 9-oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht, und der gegebenenfalls wie ($C_6$-$C_{14}$)-Aryl unter $a_1$) definiert, mono-, di- oder trisubstituiert ist, bedeutet
oder
$a_2$) einen Rest der Formel VII bedeutet

$R^{1'}$ - W (VII)

worin $R^{1'}$ wie $R^1$ unter $a_1$) definiert ist und W für -CO-, -O-CO-, $-SO_2-$, -SO-, $-HN-SO_2-$, HN-CO-, -CH(OH)- oder -N(OH)- steht,
$R^2$, $R^5$ und $R^9$ unabhängig voneinander wie $R^1$ unter $a_1$) definiert sind,
$R^3$ und $R^4$ unabhängig voneinander Wasserstoff oder ($C_1$-$C_{12}$)-Alkyl bedeuten, wobei Aryl gegebenenfalls ein-bis zweifach ungesättigt ist und gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist; oder zusammen mit Bor, X und Y ein mono-, bi- oder tricyclisches, gesättigtes oder teilweise ungesättigtes mono-, di-, tri- oder tetra-($C_1$-$C_{12}$)-alkyliertes Ringsystem mit 5-18 Ringgliedern, das außer Bor, X, Y und Kohlenstoff noch ein -O- Glied, ein $-NR^{13}$-Glied oder ein $-CR^{14}R^{15}$-Glied enthalten kann, bilden,
X und Y unabhängig voneinander für -O- oder $-NR^{13}-$ stehen,
$R^6$ für Wasserstoff oder ($C_1$-$C_8$)-Alkyl steht, oder zusammen mit $R^5$ ein mono- oder bicyclisches, gesättigtes oder teilweise ungesättigtes Ringsystem mit 5-12 Ringgliedern bildet,
$R^7$ und $R^8$ unabhängig voneinander Wasserstoff; Hydroxy; Amino; Fluor; Amino-($C_1$-$C_4$)-alkyl; Hydroxy-($C_1$-$C_4$)-alkyl; ($C_1$-$C_4$)-Alkyl, das auch einfach ungesättigt sein kann, bedeuten,
$R^{10}$ und $R^{11}$ unabhängig voneinander Wasserstoff, Hydroxy oder ($C_6$-$C_{14}$)-Aryl bedeuten, wobei Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5-oder 6-gliedrigen monocyclischen oder 9-oder 10-gliedrigen bicyclischen, gegebenenfalls teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder stehen, der gegebenenfalls wie oben ($C_6$-$C_{14}$)-Aryl mono- oder disubstituiert ist,
n und m unabhängig voneinander 0, 1, 2, 3 und 4 sein können,
$R^{12}$ Wasserstoff oder ($C_1$-$C_8$)-Alkyl ist, oder gemeinsam mit $R^1$ ein mono- oder bicyclisches, gesättigtes oder teilweise ungesättigtes Ringsystem mit 5 - 12 Ringgliedern bildet, das außer Kohlenstoff noch 1 Schwefelatom enthalten kann, welches gegebenenfalls zum Sulfoxid oder Sulfon oxidiert sein kann,
$A^2$ entweder abwesend ist, wobei gleichzeitig $A^1$ nicht für einen Rest der Formel II steht, oder einen N-terminal mit $A^1$ und C-terminal mit dem Aminoboronsäurederivat verknüpften Rest der Formel VIII bedeutet,

```
  R6   R5   O
  |    |    ||
- N  - CH - C -        (VIII)
```

wobei $R^5$ und $R^6$ wie oben definiert sind,
$R^{13}$ Wasserstoff oder ($C_1$-$C_{12}$)-Alkyl, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, bedeutet,
$R^{14}$ und $R^{15}$ unabhängig voneinander Wasserstoff, ($C_1$-$C_8$)-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)amino, Bis(2-hydroxyethyl)amino bedeuten
sowie deren physiologisch verträgliche Salze, wobei die Verbindungen Boc-Phe-Pro-[Benzyl, (4,4,5,5-tetramethyl-1,3,2-dioxaborolan-2-yl)]methylamid, Boc-Phe-Gly-[Isobutyl,(4,4,5,5-tetramethyl-1,3,2-dioxaborolan-2-yl)]methylamid, Boc-Phe-Gly-[Isobutyl,[(N-B)-(2,2′-iminodiethanolato)boryl]]methylamid, H-Phe-Gly-[Isobutyl,(4,4,5,5-tetramethyl-1,3,2-dioxaborolan-2-yl)]methylamid Trifluoracetat und Boc-D-Phe-Pro[Isopropyl,(4,4,5,5-tetramethyl-1,3,2-dioxaborolan-2-yl)]methylamid ausgenommen sind.

Unter einem Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 - 2 S-Atomen und/oder 1 - 2 O-Atomen als Ringglieder werden Reste von Heteroaromaten verstanden, wie sie beispielsweise in Katritzky, Lagowski, Chemie der Heterocyclen, Berlin, Heidelberg 1968 definiert sind. Der Heteroaromaten-Rest kann durch einen, zwei oder drei, vorzugsweise einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert sein Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. ganz oder teilweise hydriertes Heteroaryl, u.a. auch z.B. Benzodioxolan, Heteroaryloxy, Heteroarylthio und Heteroaryl-alkyl. Alkyl kann geradkettig oder verzweigt sein. Entsprechendes gilt für davon abgeleitete Reste, wie z.B. Alkoxy, Alkylthio, Alkylamino, Dialkylamino, Alkylsulfinyl, Alkylsulfonyl, Alkanoyl und Aralkyl.

($C_6$-$C_{14}$)-Aryl ist beispielsweise Phenyl, Naphthyl, Biphenylyl oder Fluorenyl; bevorzugt sind Phenyl und Naphthyl. Entsprechendes gilt für davon abgeleitete Reste, wie z.B. Aryloxy, Aroyl, Aralkyl und Aralkyloxy. Unter Aralkyl versteht man einen mit ($C_1$-$C_6$)-Alkyl verknüpften unsubstituierten oder substituierten ($C_6$-$C_{14}$)-Aryl-Rest, wie z.B. Benzyl, 1- und 2-Naphthylmethyl, Halobenzyl und Alkoxybenzyl, wobei Aralkyl jedoch nicht auf die genannten Reste beschränkt wäre.

Unter Salzen von Verbindungen der Formel I sind insbesondere pharmazeutisch verwendbare oder nichttoxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in denen die Reste wie folgt definiert sind:
$A^1$ ist wie auf der Seite 2 definiert,
$R^1$ bedeutet vorzugsweise Wasserstoff oder steht für ($C_1$-$C_{10}$)-Alkyl; Cyclopentyl; Cyclohexyl; Cyclopentyl-($C_1$-$C_{10}$)-alkyl; Cyclohexyl-($C_1$-$C_{10}$)-alkyl; gegebenenfalls substituiertes Phenyl-($C_1$-$C_8$)-alkyl; 2-Pyridyl-($C_1$-$C_8$)-alkyl; 3-Pyridyl-($C_1$-$C_8$)-alkyl; 4-Pyridyl-($C_1$-$C_8$)-alkyl; $H_2N$-($C_1$-$C_{10}$)-Alkyl; HO-($C_1$-$C_{10}$)-Alkyl; ($C_1$-$C_4$)-Alkoxy-($C_1$-$C_{10}$)-alkyl; ($C_1$-$C_4$)-Alkoxycarbonyl-($C_1$-$C_{10}$)-alkyl; ($C_1$-$C_8$)-Alkylsulfonyl; ($C_1$-$C_8$)-Alkylsulfinyl; Hydroxy-($C_1$-$C_{10}$)-alkanoyl, wie 2-Hydroxypropionyl oder 2-Hydroxy-3-methylbutyryl; ($C_1$-$C_8$)-Alkanoyloxy-($C_1$-$C_{10}$)-alkyl; ($C_1$-$C_{11}$)-Alkanoyl, wie n-Decanoyl, Formyl, Acetyl, Propionyl, Pivaloyl, Isovaleryl oder Isobutyryl; gegebenenfalls geschütztes Amino-($C_1$-$C_{11}$)-alkanoyl, wie (3-Amino,3,3-dimethyl)propionyl, 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl; Di-($C_1$-$C_7$)-alkylamino-($C_2$-$C_{11}$)-alkanoyl, wie Dimethylaminoacetyl; ($C_3$-$C_9$)-Cycloalkylcarbonyl, wie Cyclopropylcarbonyl, Cyclobutylcarbonyl, Cyclopentylcarbonyl oder Cyclohexylcarbonyl; ($C_6$-$C_{10}$)-Aryl-($C_2$-$C_{11}$)-alkanoyl, wie Phenylacetyl, Phenylpropanoyl oder Phenylbutanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl; Pyrrolyl-2-carbonyl; Pyridyl-3-carbonyl; Benzolsulfonyl; ($C_1$-$C_{10}$)-Alkoxycarbonyl, wie Methoxycarbonyl, Ethoxycarbonyl, Isobutoxycarbonyl oder tert.-Butoxycarbonyl; substituiertes ($C_1$-$C_{10}$)-Alkoxycarbonyl, wie 2-(Trimethylsilyl)ethoxycarbonyl, 2,2,2-Trichlorethoxycarbonyl oder 1,1-Dimethyl-2,2,2-trichlorethoxycarbonyl; ($C_6$-$C_{14}$)-Aryl-($C_1$-$C_6$)-alkoxycarbonyl, wie Benzyloxycarbonyl, 1- oder 2-Naphthylmethoxycarbonyl oder 9-Fluorenylmethoxycarbonyl; oder $R^1$ bildet bevorzugt gemeinsam mit $R^{12}$ ein mono- oder bicyclisches, gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxydiert sein kann,
$R^2$ ist vorzugsweise ($C_3$-$C_{12}$)-Alkyl; mono-, bi- oder tricyclisches ($C_3$-$C_{18}$)-Cycloalkyl, ($C_1$-$C_{18}$)-Cycloalkylmethyl, ($C_3$-$C_{18}$)-Cycloalkylethyl, wobei der Cycloalkylteil gegebenenfalls durch ($C_1$-$C_6$)-Alkyl substituiert ist; Dithiolanyl; ($C_6$-$C_{14}$)-Arylmethyl; Dithiolanylmethyl; Dithiolanylethyl; Dithianyl; Dithianylmethyl oder Dithianylethyl,
$R^3$ und $R^4$ sind vorzugsweise unabhängig voneinander Wasserstoff, ($C_1$-$C_{12}$)-Alkyl oder bilden zusammen mit Bor, X und Y ein mono-, bi- oder tricyclisches, gesättigtes oder teilweise ungesättigtes, gegebenenfalls mono-, di-, tri- oder tetra-($C_1$-$C_{12}$)-alkyliertes Ringsystem mit 5-18 Ringgliedern, das außer Bor, X, Y und

Kohlenstoff noch ein -O- Glied, ein -$NR^{13}$-Glied oder ein -$CR^{14}R^{15}$-Glied enthalten kann,
X und Y sind unabhängig voneinander bevorzugt -O- oder -$NR^{13}$-,
$R^5$ und $R^9$ sind unabhängig voneinander bevorzugt Wasserstoff; ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, Amidino, Guanidino, Carbamoyl, ($C_1$-$C_5$)-Alkoxycarbonylamino, ($C_7$-$C_{15}$)-Aralkoxycarbonylamino und 9-Fluorenylmethoxycarbonylamino substituiert ist; ($C_3$-$C_{12}$)-Cycloalkyl-($C_1$-$C_3$)-alkyl, mono- oder bicyclisches ($C_6$-$C_{14}$)-Aryl -($C_1$-$C_3$)-Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist; oder stehen bevorzugt für ($C_1$-$C_3$)-Alkyl, substituiert mit dem Rest eines 5- oder 6-gliedrigen monocyclischen oder 9- oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie auf Seite 4 für ($C_6$-$C_{14}$)-Aryl beschrieben mono- oder disubstituiert ist,
$R^6$ ist bevorzugt Wasserstoff, Methyl oder Ethyl oder bildet zusammen mit $R^5$ bevorzugt Pyrrolidin oder Piperidin, die jeweils noch mit Cyclopentyl, Cyclohexyl oder Phenyl anneliert sein können,
$R^7$ und $R^8$ sind unabhängig voneinander bevorzugt Wasserstoff, Hydroxy, Amino, Fluor, Hydroxymethyl oder Aminomethyl,
$R^{10}$ und $R^{11}$ sind unabhängig voneinander bevorzugt Wasserstoff, Hydroxy, Phenyl, 2- oder 3-Thienyl, 2-, 3-oder 4-Pyridyl, 1-, 2- oder 4-Imidazolyl, 1- oder 2-Naphthyl; 2- oder 3-Benzo[b]thienyl,
n und m können unabhängig voneinander 0, 1, 2, 3 und 4 bedeuten,
$R^{12}$ ist wie auf Seite 5 definiert,
$R^{13}$ ist bevorzugt Wasserstoff oder ($C_1$-$C_{12}$)-Alkyl,
$R^{14}$ und $R^{15}$ sind wie auf Seite 6 definiert,
$A^2$ ist abwesend, wobei gleichzeitig $A^1$ nicht für einen Rest der Formel II steht, oder bedeutet einen Rest der Formel VIII, wobei $R^5$ und $R^6$ wie auf den Seiten 9 und 10 definiert sind.

Besonders bevorzugt sind Verbindungen der Formel I, in denen die Reste wie folgt definiert sind:
$A^1$ ist wie auf der Seite 2 definiert,
$R^1$ bedeutet besonders bevorzugt ($C_1$-$C_8$)-Alkylsulfonyl; ($C_1$-$C_8$)-Alkylsulfinyl; Hydroxy-($C_1$-$C_{10}$)-alkanoyl, wie 2-Hydroxypropionyl oder 2-Hydroxy-3-methylbutyryl; ($C_1$-$C_8$)-Alkanoyloxy-($C_1$-$C_{10}$)-alkyl; ($C_1$-$C_{11}$)Alkanoyl, wie n-Decanoyl, Formyl, Acetyl, Propionyl, Pivaloyl, Isovaleryl oder Isobutyryl; Amino-($C_1$-$C_{11}$)-alkanoyl, wie (3 -Amino, 3,3-dimethyl)propionyl , 4-Aminobutyryl, 5-Aminopentanoyl, 6-Aminohexanoyl; Di-($C_1$-$C_7$)-alkylamino-($C_2$-$C_{11}$)-alkanoyl, wie Dimethylaminoacetyl; ($C_3$-$C_9$)-Cycloalkylcarbonyl, wie Cyclopropylcarbonyl, Cyclobutylcarbonyl, Cyclopentylcarbonyl oder Cyclohexylcarbonyl; ($C_6$-$C_{10}$)-Aryl-($C_2$-$C_{11}$)-alkanoyl, wie Phenylacetyl, Phenylpropanoyl oder Phenylbutanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl; Pyrrolyl-2-carbonyl; Pyridyl-3-carbonyl; Benzolsulfonyl; ($C_1$-$C_{10}$)-Alkoxycarbonyl, wie Methoxycarbonyl, Ethoxycarbonyl, Isobutoxycarbonyl oder tert.-Butoxycarbonyl; substituiertes ($C_1$-$C_{10}$)-Alkoxycarbonyl, wie 2-(Trimethylsilyl)-ethoxycarbonyl, 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, wie Benzyloxycarbonyl, 1- oder 2-Naphthylmethoxycarbonyl oder 9-Fluorenylmethoxycarbonyl,
$R^2$ ist besonders bevorzugt ($C_3$-$C_{12}$)-Alkyl; mono-, bi- oder tricyclisches ($C_3$-$C_{18}$)-Cycloalkyl oder ($C_3$-$C_{18}$)-Cycloalkylmethyl, wobei der Cycloalkylteil gegebenenfalls durch ($C_1$-$C_4$)-Alkyl substituiert ist; ($C_6$-$C_{14}$)-Arylmethyl; Dithiolanyl; Dithiolanylmethyl; Dithianyl und Dithianylmethyl,
$R^3$ und $R^4$ sind besonders bevorzugt Wasserstoff oder bilden gemeinsam mit Bor, X und Y ein mono-, bi- oder tricyclisches mono-, di-, tri- oder tetra-($C_1$-$C_{12}$)-alkyliertes, gesättigtes oder teilweise ungesättigtes Ringsystem mit 5-18 Ringgliedern, das außer Bor, X, Y und Kohlenstoff noch ein -O- Glied, ein -$NR^{13}$-Glied oder ein -$CR^{14}R^{15}$-Glied enthalten kann,
X und Y stehen besonders bevorzugt für -O-,
$R^5$ und $R^9$ sind unabhängig voneinander besonders bevorzugt Wasserstoff, Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto,1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)ethyl, 3-Thienyl, 3-Thienylmethyl, 2-(3-Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methyl-imidazol-4-yl)methyl, (3-Methyl-Imidazol-4-yl)methyl, Phenyl, 1-Naphthylmethyl, 2-Naphthylmethyl, 2-Phenylmethyl, 2-Thiazolylmethyl, 4-Thiazolylmethyl, 3-Pyrazolylmethyl, 4-Pyrimidinylmethyl, Indol-2-yl-methyl, 2-Benzo[b]thienylmethyl, 3-Benzo[b]thienylmethyl, 2-Furylmethyl,
$R^6$ bedeutet besonders bevorzugt Wasserstoff oder Methyl oder bildet gemeinsam mit $R^5$ und der diese Reste tragenden -N-CH-Gruppe ein Tetrahydroisochinolin-oder Azabicyclooctan-Gerüst,
einer der Reste $R^7$ oder $R^8$ bedeutet besonders bevorzugt Wasserstoff, der jeweils andere Rest Hydroxy, Amino, Fluor, Hydroxymethyl oder Aminomethyl,

$R^{10}$ und $R^{11}$ sind unabhängig voneinander besonders bevorzugt Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl,

n und m sind unabhängig voneinander besonders bevorzugt 0, 1 oder 2,

$R^{12}$ ist besonders bevorzugt Wasserstoff oder Methyl, oder bildet gemeinsam mit $R^1$ ein mono- oder bicyclisches gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxidiert sein kann,

$R^{13}$ ist besonders bevorzugt Wasserstoff oder $(C_1-C_4)$-Alkyl,

$R^{14}$ und $R^{15}$ sind wie auf Seite 6 definiert,

$A^2$ ist abwesend, wobei gleichzeitig $A^1$ nicht für einen Rest der Formel II steht, oder bedeutet einen Rest der Formel VIII, wobei $R^5$ und $R^6$ wie auf der Seite 4 definiert ist.

Ferner sind besonders bevorzugt Verbindungen der Formel I, in denen

$A^1$ für einen Rest der Formel II, III oder VI steht, worin

$R^1$ $(C_1-C_6)$-Alkylsulfonyl, wie z.B. Ethylsulfonyl, Isopropylsulfonyl, Isobutylsulfonyl, sec.-Butylsulfonyl oder tert.-Butylsulfonyl; $(C_1-C_6)$-Alkylsulfinyl, wie z.B. Ethylsulfinyl, Isopropylsulfinyl, Isobutylsulfinyl, sec.-Butylsulfinyl oder tert.-Butylsulfinyl; $(C_1-C_6)$-Alkanoyl, wie z.B. Acetyl, Propionyl, Isobutyryl, Isovaleryl oder Pivaloyl; Amino-$(C_1-C_6)$-alkanoyl, wie z.B. (3-Amino,3,3-dimethyl)propionyl; $(C_1-C_6)$-Alkoxycarbonyl, wie z.B. Ethoxycarbonyl, Isobutoxycarbonyl oder tert.-Butoxycarbonyl; $(C_6-C_{10})$-Aryl-$(C_1-C_6)$-alkoxycarbonyl, wie z.B. Benzyloxycarbonyl, 1- oder 2-Naphthylmethoxycarbonyl bedeutet,

$R^2$ $(C_5-C_8)$-Cycloalkyl; $(C_5-C_{11})$-Cycloalkylmethyl; $[(C_1-C_4)$-Alkyl-cyclohexyl]methyl; $(C_3-C_4)$-Alkyl, das durch $(C_1-C_3)$-Alkyl substituiert ist; $(C_6-C_{10})$-Arylmethyl oder Dithiolan-2-yl-methyl bedeutet,

$R^3$ und $R^4$ für Wasserstoff stehen oder zusammen mit Bor, X und Y einen 1,3,2-Dioxaborolan-Rest, der gegebenenfalls durch 1 bis 4 Methylgruppen substituiert ist, einen [4,5-Diisopropyl]-1,3,2-dioxaborolan-, einen [(N-B)-(2,2′-imino-diethanolato)-boryl)]-, einen Pinandioxyboryl- oder einen 1,3,2-Dioxaborinan-Rest, dessen C-Atom in Position 5 durch $R^{14}$ und $R^{15}$ substituiert ist, bilden,

X und Y bevorzugt für -O- stehen,

$R^5$ Wasserstoff, n-Propyl, Isopropyl, n-Butyl, Isobutyl, sec.-Butyl, Carbamoylmethyl, 2-Carbamoylethyl, Benzyl, 2-Methylbenzyl, 3-Methylbenzyl, 2-Thienylmethyl, 3-Thienylmethyl, 1-Naphthylmethyl, 2-Naphthylmethyl, Imidazol-4-yl-methyl oder 2-Thiazolylmethyl bedeutet,

$R^6$ für Wasserstoff oder Methyl steht,

$R^{10}$ und $R^{11}$ unabhängig voneinander 1-Naphthyl oder 2-Thienyl bedeuten,

n und m für 1 stehen,

$R^{12}$ Wasserstoff bedeutet,

$R^{14}$ und $R^{15}$ unabhängig voneinander Wasserstoff, $(C_1-C_8)$-Alkyl, Hydroxymethyl, 2-Hydroxyethyl (3-Hydroxysulfonyl, 2-hydroxypropyl)amino, (2-Hydroxysulfonylethyl)amino, (2-Hydroxysulfonylpropyl)amino, (Carboxymethyl)amino oder Bis(2-hydroxyethyl)amino bedeuten,

und

$A^2$ für einen Rest der Formel (VIII steht, wobei $R^5$ und $R^6$ wie auf den Seiten 12 und 13 definiert sind.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln IX bis XI

$A^1$ - OH (IX)

$A^1$ - $A^2$ - OH (X)

Fragmente einer Verbindung der Formel I mit einer endständigen Aminogruppe besitzen die nachstehenden Formeln XI bis XIV

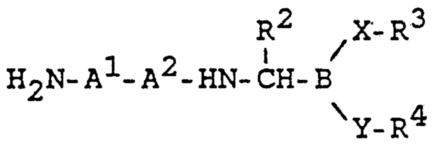

(XI)

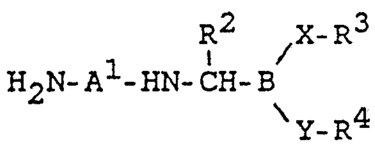

(XII)

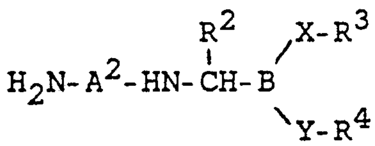

(XIII)

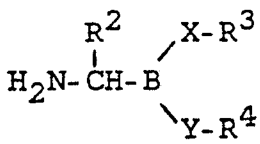

(XIV)

Methoden, die zur Herstellung einer Amidbindung geeignet sind, werden z.B. in Houben-Weyl, Methoden der organischen Chemie, Band 15/2; Bodanszky et al., Peptide Synthesis, 2nd ed. (Wiley & Sons, New York 1976) oder Gross, Meienhofer, The Peptides: Analysis, synthesis, biology (Academic Press, New York 1979) beschrieben. Vorzugsweise werden die folgenden Methoden herangezogen:
Aktivestermethode mit N-Hydroxy-succinimid, 1-Hydroxybenzotriazol oder 3-Hydroxy-4-oxo-3,4-dihydro-1,2,3-benzotriazin als Alkoholkomponente, Kupplung mit einem Carbodiimid wie Dicyclohexylcarbodiimid oder mit Propanphosphonsäureanhydrid und die Gemischt-Anhydrid-Methode mit Pivaloylchlorid oder Chlorameisensäureethylester oder -isobutylester.

Fragmente der Formel IX, sofern sie unter

a) Formel II fallen, werden nach den allgemein bekannten Methoden zur Herstellung von Aminosäuren synthetisiert;

b) Formel III fallen, werden ausgehend von den entsprechenden Aminosäuren synthetisiert, wobei deren Chiralitätszentrum erhalten bleibt. Diazotierung bei -20° C bis 50° C in verd. Mineralsäuren führt zu α-Bromcarbonsäuren oder über die Milchsäuren zu α-Trifluormethansulfonyloxy-Carbonsäuren, die mit einem $R^1$ und $R^{12}$ tragenden Nucleophil umgesetzt werden können;

c) Formel IV fallen, werden ausgehend von Aminoaldehyden, die nach B. Castro et al. (Synthesis 1983, 676) hergestellt werden, synthetisiert. Wittig-Reaktion mit Methyltriphenylphosphoniumbromid unter den allgemein bekannten Bedingungen und anschließende Epoxidierung mit m-Chlorperbenzoesäure liefert N-geschützte Aminoepoxide. Öffnung mit Trimethylsilylchlorid/NaI in Acetonitril im Temperaturbereich von -20°C bis zum Siedepunkt des Lösungsmittels, Desilylierung mit CsF und Acetonierung mit 2,2-Dimethoxypropan/p-Toluolsulfonsäure bei 0° C bis 110° C liefert ein geschütztes Jodid. Dieses wird entweder direkt mit Esterenolaten nach den allgemein bekannten Bedingungen der C-Alkylierung von Esterenolaten umgesetzt oder, wenn höhere Reaktivität gewünscht ist, über das Formiat (hergestellt mit Na-Formiat in einem dipolar aprotischen Lösungsmittel bei 60° C bis 150° C) und den Alkohol in das Trifluormethansulfonat überführt. Mit diesem Elektrophil lassen sich auch Esterenolate umsetzen, die aufgrund sterischer Hinderung in $R^9$ sich nicht wie oben beschreiben mit dem Jodid umsetzen lassen;

d) Formel V fallen, werden ausgehend von der entsprechenden Carbonsäure durch Umsetzung mit einem Rest der Formel III hergestellt und nach der Überführung in die Aldehyde wie unter c) beschrieben weiter umgesetzt;

e) Formel VI fallen, werden ausgehend von Malonestern hergestellt, deren Alkylierung mit Arylalkylhalogeniden mono- oder disubstituierte Malonester liefert, die nach Verseifung durch Decarboxylierung in die gewünschten Derivate überführt werden. Für den Fall, daß einer der Reste $R^{10}$ oder $R^{11}$ Hydroxy bedeutet, geht man von der entsprechenden Aminosäure aus und erhält nach Diazotierung (wie oben beschrieben) die Milchsäure (Anzahl der $R^{10}$ oder $R^{11}$ tragenden $CH_2$-Gruppen = 0) oder man geht von der substituierten Malonsäure aus, wobei Monoverseifung und selektive Reduktion (mit z.B. Diboran oder $LiAlH_4$) die 2-substituierte 3-Hydroxy-propionsäure ergibt.

Die Aminoboronsäurederivate aus Formel I werden ausgehend von Borsäuretrialkylestern wie z.B. $B(OMe)_3$, $B(OEt)_3$, $B(OiPr)_3$, $B(OnBu)_3$ oder $B(OtBu)_3$ hergestellt. Die Einführung von $R^2$ erfolgt über eine Grignard-Reaktion bei Temperaturen zwischen -100°C bis +50°C, bevorzugt zwischen -78°C und 0°C in einem gegenüber metallorganischen Reagentien inerten Lösungsmittel wie Ethern, so zum Beispiel Diethylether, Tetrahydrofuran oder Dimethoxyethan. Unter gleichen Reaktionsbedingungen können auch entsprechende Alkyllithium- oder Aryllithiumverbindungen zur Einführung von $R^2$ dienen. Anschließend erfolgt zweckmäßigerweise die Überführung in einen relativ hydrolysebeständigen Boronsäureester wie den Pinakolester oder, wenn die Synthese asymmetrisch gestaltet werden soll in den Pinandiolester gewünschter Stereochemie oder den 1,2-Diisopropylethandiol-Ester. Dies erfolgt am Besten unter Säurekatalyse und Entfernung des Niederalkyl-Alkohols unter vermindertem Druck. Anschließend werden die Boronsäureester mit $LiCHCl_2$ bei -120°C bis -70°C, bevorzugt bei -110°C bis -90°C in einem Lösungsmittel wie Diethylether oder Tetrahydrofuran umgesetzt. Zunächst entsteht das Dichlormethylboronat, das schließlich bei Raumtemperatur unter $Cl^-$-Eliminierung in den um eine (-CHCl-)-Gruppe verlängerten Boronsäureester umlagert. Zur Erhöhung der Reaktivität wird Chlor noch durch Jod substituiert durch Umsetzung mit NaI in Acetonitril bei 0°C bis 50°C. Dessen Umsetzung mit Lithium-bis-(trimethylsilyl)amid bei 0°C bis 50°C in einem gegenüber starken Basen inerten Lösungsmittel wie Ethern und anschließende Desilylierung des Produkts in Trifluoressigsäure liefert die substituierten α-Aminoboronsäureester als kristallisierende Trifluoressigsäuresalze. Diese können analog der Peptidkupplung mit Aminocarbonsäureestern mit den Resten $A^1$-OH oder $A^1$-$A^2$-OH umgesetzt werden.

Die zur Herstellung von Verbindungen der Formel I erforderlichen Vor- und Nachoperationen wie Einführung und Abspaltung von Schutzgruppen sind literaturbekannt und sind z.B. in T.W. Greene "Protective Groups in Organic Synthesis" (John Wiley & Sons, New York, 1981) beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die gegebenenfalls bei der Synthese von Verbindungen der Formel I anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin 1 ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkonstriktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37°C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4°C, 15 min; wiederholen, falls erforderlich) wird das Plasma vorsichtig abpipettiert und in geeigneten Portionen bei -30°C eingefroren. Für den Test werden nur Plasmen mit ausreichend hoher Reninaktivität verwendet. Plasmen mit niedriger Reninaktivität werden durch eine Kältebehandlung (-4°C, 3 Tage aktiviert (Prorenin → Renin).

**4. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:

Inkubationsansatz: 1000 µl Plasma (bei 0-4°C aufgetaut)

100 µl Phosphatpuffer (pH 7,4) Zusatz von $10^{-4}$ M Ramiprilat)

10 µl PMSF-Lösung

10 µl 0,1 % Genapol PFIC

12 µl DMSO bzw. Testpräparat

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37° C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25° C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in dem in-vitro-Test Hemmwirkungen bei Konzentrationen von etwa $10^{-5}$ bis $10^{-10}$ Mol/l. Im einzelnen wurden folgende Werte ermittelt:

**Tabelle 1:**

| Beispiel-Nr. | $IC_{50}$[M] humanes Plasmarenin |
|---|---|
| 1 | $4{,}2\cdot10^{-7}$ |
| 2 | $1{,}0\cdot10^{-6}$ |
| 3 | $2{,}0\cdot10^{-6}$ |
| 4 | $4{,}5\cdot10^{-7}$ |
| 5 | $5{,}5\cdot10^{-7}$ |
| 7 | $6{,}7\cdot10^{-7}$ |
| 8 | $6{,}5\cdot10^{-7}$ |
| 9 | $2{,}7\cdot10^{-7}$ |
| 13 | $2{,}4\cdot10^{-6}$ |
| 15 | $2{,}8\cdot10^{-6}$ |
| 16 | $3{,}0\cdot10^{-6}$ |
| 18 | $9{,}0\cdot10^{-6}$ |
| 19 | $8{,}0\cdot10^{-6}$ |
| 22 | $7{,}3\cdot10^{-6}$ |
| 23 | $7{,}8\cdot10^{-6}$ |
| 29 | $4{,}4\cdot10^{-6}$ |
| 30 | $1{,}7\cdot10^{-6}$ |
| 31 | $3{,}3\cdot10^{-7}$ |
| 32 | $5{,}0\cdot10^{-7}$ |
| 33 | $5{,}0\cdot10^{-6}$ |
| 34 | $3{,}0\cdot10^{-6}$ |
| 35 | $2{,}8\cdot10^{-7}$ |
| 36 | $6{,}2\cdot10^{-7}$ |
| 39 | $2{,}1\cdot10^{-6}$ |

Tabelle 1: Fortsetzung

| Beispiel-Nr. | $IC_{50}$[M] humanes Plasmarenin |
|---|---|
| 40 | $3{,}6 \cdot 10^{-6}$ |
| 41 | $1{,}5 \cdot 10^{-6}$ |
| 42 | $9{,}5 \cdot 10^{-7}$ |
| 43 | $5{,}5 \cdot 10^{-7}$ |
| 44 | $2{,}4 \cdot 10^{-6}$ |
| 50 | $1{,}5 \cdot 10^{-6}$ |
| 51 | $1{,}8 \cdot 10^{-6}$ |
| 52 | $2{,}8 \cdot 10^{-6}$ |
| 54 | $9{,}0 \cdot 10^{-7}$ |

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten, wie zum Beispiel Rhesus-Affen, herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1 - 50 mg/kg. Die in der vorliegenden Erfindung beschriebenen Verbindungen der allgemeinen Formel I können als Antihypertensiva sowie zur Behandlung der Herzinsuffizienz verwendet werden.

Die Erfindung betrifft daher auch die Verwendung von Verbindungen der Formel I als Heilmittel und pharmazeutische Präparate, die diese Verbindungen enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischen oder organischen pharmazeutisch verwendbaren Trägerstoff.

Die Anwendung kann intranasal, intravenös, subkutan, peroral oder intraduodenal erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

**Verzeichnis der verwendeten Abkürzungen:**

| | |
|---|---|
| Ac | Acetyl |
| Boc | tert.-Butoxycarbonyl |
| BuLi | n-Butyllithium |
| DC | Dünnschichtchromatographie |
| DCC | Dicyclohexylcarbodiimid |
| DCI | Desorption Chemical Ionisation |
| DIP | Diisopropylether |
| DNP | 2,4-Dinitrophenyl |
| DMF | Dimethylformamid |
| DMSO | Dimethylsulfoxid |
| EE | Essigsäureethylester |
| EI | Electron Impact |
| Etoc | Ethoxycarbonyl |
| FAB | Fast atom bombardment |
| H | Hexan |
| HOBt | 1-Hydroxybenzotriazol |
| Iva | Isovaleryl |
| M | Molekularpeak |
| MeOH | Methanol |
| MS | Massenspektrum |
| MTB | Methyl-tert.-butylether |
| Nva | Norvalin |
| Nle | Norleucin |
| R.T. | Raumtemperatur |
| Schmp. | Schmelzpunkt |
| $Sdp_{xx}$ | Siedepunkt bei xx Torr |
| Thi | β-2-Thienylalanin |
| THF | Tetrahydrofuran |
| Z | Benzyloxycarbonyl. |

Die sonstigen für Aminosäuren verwendeten Abkürzungen entsprechen dem in der Peptidchemie üblichem drei Buchstaben-Code wie er z.B. in Europ. J. Biochem. 138, 9 - 37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

**Beispiel 1**

Iva-Phe-Nva-[Cyclohexylmethyl,(4,4,5,5-tetramethyl-1,3,2-dioxaborolan-2-yl)]methylamid

139 mg Iva-Phe-Nva-OH werden in 5 ml THF gelöst und bei -20°C erst 44 µl N-Methylmorpholin, dann 52 µl Chlorameisensäureisobutylester zugespritzt. Nach 5 Minuten bei -20°C wird 56 µl Triethylamin in 2 ml THF addiert. Das so erhaltene gemischte Anhydrid wird bei -20°C zu einer Lösung von 2-[(1′-Amino-2′-cyclohexyl)ethyl],4,4,5,5-tetramethyl-1,3,2-dioxaborolan (Trifluoressigsäuresalz) in 5 ml THF gespritzt, bei -20°C 1 h, bei R.T. weitere 2 h gerührt, THF im Vakuum entfernt und in 20 ml Essigester aufgenommen. 2 mal wird mit 20 ml gesättigter wäßruger $NaHCO_3$, 2 mal mit 20 ml 0,06 M $KH_2PO_4$ extrahiert. Anschließend wird über $Na_2SO_4$ getrocknet, das Solvens im Vakuum entfernt und an Kieselgel mit MTB chromatographiert. Man erhält 105 mg der Titelverbindung als farbloses amorphes Pulver.

$R_f$(MTB) = 0,38 MS(FAB): 584 (M + 1)

a) 2-[(1′-Amino-2′-cyclohexyl)ethyl],4,4,5,5-tetramethyl-1,3,2-dioxaborolan (Trifluoressigsäuresalz)

0,7 ml Hexamethyldisilazan werden in 8 ml THF gelöst und bei -78°C mit 2,1 ml 1,6 M BuLi in Hexan versetzt. Man läßt 5 Minuten bei R.T. rühren, kühlt erneut auf -78°C ab und gibt 1,1 g 2-[(1′-Jod-2′-cyclohexyl)ethyl]4,4,5,5-tetramethyl-1,3,2-dioxaborolan in 5 ml THF zu. 22 h wird bei R.T. gerührt, dann das THF im Vakuum entfernt und der Rückstand in 4 ml Diethylether aufgenommen. Anschließend wird bei 0°C 0,3 ml Trifluoressigsäure langsam zugetropft und 10 Minuten bei dieser Temperatur nachgerührt, wobei ein weißer kristalliner Niederschlag ausfällt. Dieser wird unter Argonatmosphäre abfiltriert und mit auf 0°C gekühltem Diethylether gewaschen. Man erhält 650 mg der Titelverbindung als weiße Kristalle.

Schmp. 154-157°C MS(DCI) : 254 (M+1)

b) 2-[(1′-Jod-2′-cyclohexyl)ethyl],4,4,5,5-tetramethyl-1,3,2-dioxaborolan

3,6 g NaI werden in 50 ml Acetonitril gelöst und 4,3 g 2-[(1′-Chlor-2′-cyclohexyl)ethyl]4,4,5,5-tetramethyl-1,3,2-dioxaborolan gelöst in 20 ml Acetonitril bei R.T. zugetropft. Nach etwa 5 Minuten beginnt NaCl auszufallen. nach 3 stündigem Rühren bei R.T. wird die Lösung abfiltriert, das Acetonitril im Vakuum entfernt und an Kieselgel mit MTB/H 1:10 chromatographiert. Man erhält 4,4 g der Titelverbindung als blaßgelbes Öl.

$R_f$ (EE/H 1:8) = 0,59 MS (EI) : 364 (M)

c) 2-[(1′-Chlor-2′-cyclohexyl)]ethyl],4,4,5,5-tetramethyl-1,3,2-dioxaborolan

6,8 g $CH_2Cl_2$ werden in 80 ml THF gelöst und auf -100° C abgekühlt. Nun wird 31,3 ml 1,6 M BuLi auf -78° C vorgekühlt und so langsam zugetropft, daß die Temperatur im Reaktionskolben nicht über -95° C ansteigt. Anschließend läßt man 11,2 g auf -78° C vorgekühltes 2-Cyclohexylmethyl,-4,4,5,5-tetramethyl-1,3,2-dioxaborolan in 25 ml Diethylether zulaufen. Anschließend wird 20 h bei R.T. gerührt, die Lösung in 300 ml Phosphatpuffer (pH = 7) eingerührt und 3 mal mit je 200 ml MTB extrahiert, über $Na_2SO_4$ getrocknet, das Solvens im Vakuum entfernt und an Kieselgel mit EE/H 1:8 chromatographiert. Man erhält 5,3 g der Titelverbindung als farbloses Öl.

$R_f$ (EE/H 1:8) = 0,50 MS (EI) : 272 (M)

d) 2-Cyclohexylmethyl,4,4,5,5-tetramethyl-1,3,2-dioxaborolan

Zu 12,0 g Magnesium und 200 ml Diethylether wird 62,9 ml Cyclohexylmethylbromid langsam zugetropft. Nach Auflösung des Magnesiums wird die Lösung der Grignard-Verbindung zu einer auf -78° C vorgekühlten Lösung von 50,3 ml Trimethylborat in 100 ml Diethylether so zugetropft, daß die Temperatur nicht über -55° C ansteigt und anschließend bei R.T. 2 h gerührt. Dann wird unter Eiskühlung 113 ml 40 %ige $H_2SO_4$ so zugegeben, daß die Innentemperatur nicht über 25° C ansteigt. Nun wird 53,2 g Pinakol in 100 ml Diethylether zugegossen und an einem Rotationsverdampfer der Diethylether sowie das Nebenprodukt Methanol entfernt. Anschließend wird mit $NaHCO_3$ auf pH = 8 eingestellt, 3 mal mit 200 ml MTB extrahiert, über $Na_2SO_4$ getrocknet, das Solvens im Vakuum entfernt und im Feinvakuum destilliert. Man erhält 49,7 g der Titelverbindung als farbloses Öl.

$Sdp_{0,05}$ = 57° C MS (EI) : 224 (M)

Die Verbindung des Beispiels 2 wird analog Beispiel 1 hergestellt.

**Beispiel 2**

Iva-Phe-Nle-[Cyclohexylmethyl, (4,4,5,5-tetramethyl-1,3,2-dioxaborolan-2-yl)]methylamid

$R_f$ (MTB) = 0,46 MS (FAB) : 598 (M + 1)

**Beispiel 3**

Iva-Phe-His-[Cyclohexylmethyl,(4,4,5,5-tetramethyl-1,3,2-dioxaborolan-2-yl)]methylamid

230 mg Iva-Phe-His(DNP)-[Cyclohexylmethyl, (4,4,5,5-tetramethyl-1,3,2-dioxaborolan-2-yl)]methylamid werden in 5 ml Acetonitril gelöst und 39 ml Thiophenol addiert. 2 h wird bei R.T. gerührt, anschließend das Solvens im Vakuum entfernt und an Kieselgel mit Aceton chromatographiert. Man erhält 65 mg der Titelverbindung als farbloses Harz.

$R_f$ (Aceton) = 0,58 MS (FAB) : 622 (M + 1)

a) Iva-Phe-His(DNP)-[Cyclohexylmethyl,(4,4,5,5-tetramethyl-1,3,2-dioxaborolan-2-yl)]methylamid wird analog Beispiel 1 hergestellt.

Die Verbindung des Beispiels 4 wird analog Beispiel 3 hergestellt.

**Beispiel 4**

Iva-Thi-His-[Cyclohexylmethyl,(4,4,5,5-tetramethyl-1,3,2-dioxaborolan-2-yl)]methylamid

$R_f$ (MTB) = 0,48 MS (FAB) : 628 (M + 1)

**Beispiel 5**

Iva-Phe-Nva-(Cyclohexylmethyl, dihydroxyboryl)methylamid

0,86 ml einer 0,5 M Lösung von $BCl_3$ in $CH_2Cl_2$ werden auf -78° C abgekühlt und 100 mg der Titelverbindung aus Beispiel 1 in 0,86 ml $CH_2Cl_2$ langsam zugetropft. 1 h wird anschließend bei 0° C gerührt, in 10 ml 0,1 N NaOH gegossen und 2 mal mit 10 ml EE gewaschen. Dann wird die wäßrige Phase mit HCl auf etwa pH = 2 angesäuert, 3 mal mit 10 ml EE extrahiert, über $Na_2SO_4$ getrocknet, das Solvens im Vakuum entfernt und an Kieselgel mit Aceton/Wasser 10 : 1 chromatographiert. Man erhält 20 mg der Titelverbindung als farblosen Schaum.

| | |
|---|---|
| $R_f$ (Aceton/$H_2O$ 10:1) = 0,45 - 0,60 | MS (FAB, Glycerinmatrix) : 557 (M+56) |

**Beispiel 6**

Iva-Phe-Nva-[Cyclohexylmethyl,[(N-B)-(2,2'-iminodiethanolato)-boryl]]methylamid

74 mg der Titelverbindung des Beispiels 1 werden in 5 ml EE suspendiert. Anschließend werden 12,2 µl Diethanolamin addiert und bei R.T. im Ultraschallbad 5 h gerührt. Das Solvens wird im Vakuum entfernt und der $CH_2Cl_2$-lösliche Teil des Reaktionsgemisches an Kieselgel mit MTB/H 5:1 chromatographiert. Man erhält 7 mg der Titelverbindung als amorphes Pulver.

$R_f$ (MTB) = 0,37

Anschließend wird die Säule mit Aceton/Wasser 10:1 eluiert und man erhält 41 mg der Titelverbindung des Beispiels 5.

Die Verbindung des Beispiels 7 wird analog Beispiel 1 hergestellt.

**Beispiel 7**

Iva-Phe-Thi-[Cyclohexylmethyl, (4,4,5,5-tetramethyl-1,3,2-dioxaborolan-2-yl)]methylamid

$R_f$ (MTB) = 0,50 MS (FAB) : 638 (M + 1)

**Beispiel 8**

Iva-Phe-Thi-(Cyclohexylmethyl,dihydroxyboryl)methylamid

165 mg der Titelverbindung aus Beispiel 7 werden in 5 ml EE gelöst und 25 µl Diethanolamin zugegeben. 5 h wird bei R.T. im Ultraschallbad gerührt, wobei der Diethanolaminester der Titelverbindung ausfällt. Das Solvens wird im Vakuum entfernt und an Kieselgel mit Aceton/$H_2O$ = 10:1 chromatographiert. Man erhält 62 mg der Titelverbindung als farblosen Schaum.

$R_f$ (Aceton/$H_2O$ 10:1) = 0,45 - 0,55

MS (FAB, Glycerinmatrix) : 611 (M + 56)

Die Verbindung des Beispiels 9 wird analog Beispiel 8 aus der Titelverbindung des Beispiels 4 hergestellt.

**Beispiel 9**

Iva-Thi-His-(Cyclohexylmethyl, dihydroxyboryl)methylamid

$R_f$ (Aceton/$H_2O$ 10:1) = 0,10

MS (FAB, Glycerinmatrix) : 601 (M + 56)

Die Verbindungen der Beispiele 10 und 11 werden analog Beispiel 1 hergestellt.

**Beispiel 10**

Iva-Phe-Nva-[(1-Naphthylmethyl),(4,4,5,5-tetramethyl-1,3,2-dioxaborolan-2-yl)]methylamid

$R_f$ (MTB) = 0,45 MS (FAB) : 628 (M + 1)

**Beispiel 11**

Iva-Phe-Nva-[Cyclooctylmethyl,(4,4,5,5-tetramethyl-1,3,2-dioxaborolan-2-yl)]methylamid

$R_f$ (MTB) = 0,43 MS (FAB) : 612 (M + 1)

Die Verbindung des Beispiels 12 wird analog Beispiel 8 aus der Titelverbindung des Beispiels 11 hergestellt.

**Beispiel 12**

Iva-Phe-Nva-(Cyclooctylmethyl, dihydroxyboryl)methylamid

$R_f$ (Aceton/$H_2O$ 10:1) = 0,55 - 0,65

MS (FAB, Glycerinmatrix) : 585 (M + 56)

Die Verbindungen der Beispiele 13-17 werden analog Beispiel 1 hergestellt.

**Beispiel 13**

Iva-Phe-Nva-[Cyclohexylmethyl, (-)-Pinandioxyboryl]-methylamid

$R_f$ (EE/H 1:1) = 0,19 MS (FAB) : 636 (M + 1)

**Beispiel 14**

Iva-Phe-Nva-[Cycloundecylmethyl,(4,4,5,5-tetramethyl-1,3,2-dioxaborolan-2-yl)]methylamid
$R_f$ (MTB) = 0,50 MS (FAB) : 550 (M + 1)

**Beispiel 15**

Iva-Phe-Nva-[(4-Methylcyclohexyl)methyl,(4,4,5,5-tetramethyl-1,3,2-dioxaborolan-2-yl)]methylamid
$R_f$ (EE/H 1:1) = 0,10 MS (FAB) : 598 (M + 1)

**Beispiel 16**

Iva-Phe-Nva-[(Cycloheptylmethyl,(4,4,5,5-tetramethyl-1,3,2-dioxaborolan-2-yl)]methylamid
$R_f$ (MTB) = 0,35 MS (FAB) : 598 (M + 1)

**Beispiel 17**

Iva-Phe-(N-Me-Phe)-[Cyclohexylmethyl,(4,4,5,5-tetramethyl-1,3,2-dioxaborolan-2-yl)]methylamid
$R_f$ (DIP/MTB 1:1) = 0,20 MS (FAB) : 646 (M + 1)

Die Verbindungen der Beispiele 18-20 werden analog Beispiel 3 hergestellt:

**Beispiel 18**

Iva-Phe-His-[Cyclooctylmethyl,(4,4,5,5-tetramethyl-1,3,2-dioxaborolan-2-yl)]methylamid
$R_f$ (Aceton) = 0,10 MS (FAB) : 650 (M + 1)

**Beispiel 19**

Iva-Phe-His-[Cycloheptylmethyl,(4,4,5,5-tetramethyl-1,3,2-dioxaborolan-2-yl)]methylamid
$R_f$ (Aceton) = 0,12 MS (FAB) : 634 (M + 1)

**Beispiel 20**

Bis-(1-Naphthylmethyl)acetyl-His-[3-methyl,1-(4,4,5,5-tetramethyl-1,3,2-dioxaborolan-2-yl)]butylamid
$R_f$ (Aceton) = 0,20 MS (FAB) : 673 (M + 1)

Die Verbindungen der Beispiele 21-34 werden aus den entsprechenden 1,3,2-Dioxaborolanen analog Beispiel 8 hergestellt.

**Beispiel 21**

Iva-Phe-Nva-(Cyclooctylmethyl,dihydroxyboryl)methylamid
$R_f$ (Aceton/$H_2O$ 10:1) = 0,54
MS (FAB, Glycerin): 586 (M+57)

**Beispiel 22**

Iva-Phe-Nva-(Cycloheptylmethyl,dihydroxyboryl)methylamid
$R_f$ (Aceton/$H_2O$ 10:1) = 0,47
MS (FAB, Glycerin): 572 (M+57)

**Beispiel 23**

Iva-Phe-Nva-[(4-Methylcyclohexyl)methyl,dihydroxyboryl]methylamid
$R_f$ (Aceton/$H_2O$ 10:1) = 0,57
MS (FAB, Glycerin): 572 (M+57)

**Beispiel 24**

Iva-Phe-His-(Cycloheptylmethyl,dihydroxyboryl)methylamid
$R_f$ (Aceton/$H_2O$ 10:1) = 0,14
MS (FAB, Glycerin): 610 (M+57)

**Beispiel 25**

Iva-Phe-His-(Cyclooctylmethyl,dihydroxyboryl)methylamid
$R_f$ (Aceton/$H_2O$ 5:1) = 0,29
MS (FAB, Glycerin): 624 (M+57)

**Beispiel 26**

Iva-Phe-Nva-(Cycloundecylmethyl,dihydroxyboryl)methylamid
$R_f$ (Aceton/$H_2O$ 15:1) = 0,45
MS (FAB, Glycerin): 628 (M+57)

**Beispiel 27**

(3-t-Butylsulfonyl)propionyl-Nva-(Cycloundecylmethyl, dihydroxyboryl)methylamid
$R_f$ (Aceton/$H_2O$ 10:1) = 0,57
MS (FAB, Glycerin): 573 (M+57)

**Beispiel 28**

(3-t-Butylsulfonyl)propionyl-Nva-(1-Naphthylmethyl, dihydroxyboryl)methylamid
$R_f$ (Aceton/$H_2O$ 10:1) = 0,29
MS (FAB, Glycerin): 547 (M+57)

**Beispiel 29**

(2-Benzyl,3-t-butylsulfonyl)propionyl-Val-(Cyclohexylmethyl,dihydroxyboryl)methylamid
$R_f$ (Aceton/$H_2O$ 10:1) = 0,57
MS (FAB, Glycerin): 593 (M+57)

**Beispiel 30**

[3-t-Butylsulfonyl,2-(2-thienylmethyl)]propionyl-Nva-(Cycloheptylmethyl,dihydroxyboryl)methylamid
$R_f$ (Aceton/$H_2O$ 10:1) = 0,55
MS (FAB, Glycerin): 613 (M+57)

**Beispiel 31**

[3-t-Butylsulfonyl,2-(2-thienylmethyl)]propionyl-Nva-(Cyclohexylmethyl,dihydroxyboryl)methylamid
$R_f$ (Aceton/$H_2O$ 10:1) = 0,44
MS (FAB, Glycerin): 599 (M+57)

**Beispiel 32**

[3-t-Butylsulfonyl,2-(2-thienylmethyl)]propionyl-Thi-(Cyclohexylmethyl,dihydroxyboryl)methylamid
$R_f$ (Aceton/$H_2O$ 20:1) = 0,35
MS (FAB, Glycerin): 653 (M+57)

**Beispiel 33**

(2-Benzyl,3-t-butylsulfonyl)propionyl-Nva-(Cyclopentylmethyl,dihydroxyboryl)methylamid
$R_f$ (Aceton/$H_2O$ 10:1) = 0,41
MS (FAB, Glycerin): 579 (M+57)

**Beispiel 34**

[3-t-Butylsulfonyl,2-(2-thienylmethyl)]propionyl-Thi-(Cycloheptylmethyl,dihydroxyboryl)methylamid
$R_f$ (Aceton/$H_2O$ 10:1) = 0,60
MS (FAB, Glycerin): 667 (M+57)

**Beispiel 35**

[3-t-Butylsulfonyl,2-(2-thienylmethyl)]propionyl-Nva-[Cyclohexylmethyl,(4,4,5,5-tetramethyl-1,3,2-dioxaborolan-2-yl)]methylamid

Die Titelverbindung wird analog Beispiel 1 aus [3-t-Butylsulfonyl,2-(2-thienylmethyl)]propionyl-Nva-OH und der Titelverbindung des Beispiels 1a hergestellt.

$R_f$ (MTB) = 0,40  MS (FAB): 625 (M+1)

a) [3-t-Butylsulfonyl,2-(2-thienylmethyl)]propionyl-Nva-OH

490 mg [3-t-Butylsulfonyl,2-(2-thienylmethyl)]propionyl-Nva-OMe werden in 4 ml Methanol gelöst, 0,4 ml $H_2O$ sowie 0,7 ml 2N NaOH zugegeben und 5h bei R.T. gerührt. Mit $NaHSO_4$-Lösung wird auf pH=2 angesäuert und 3 x mit 50 ml EE extrahiert. Anschließend wird über $Na_2SO_4$ getrocknet und das Solvens im Vakuum entfernt. Man erhält 470 mg blaßgelbes Harz.

$R_f$ (EE) = 0,24  MS (FAB): 390 (M+1)

b) [3-t-Butylsulfonyl,2-(2-thienylmethyl)]propionyl-Nva-OMe

1,5 g [3-t-Butylsulfonyl,2-(2-thienylmethyl)]propionsäure sowie 0,95 g Nva-OMe x HCl werden in 40 ml $CH_2Cl_2$ gelöst und bei 10°C zunächst 3,8 ml Triethylamin, dann 3,5 ml einer 50 % Lösung von Propanphosphonsäure-Anhydrid in $CH_2Cl_2$ zugegeben. 20 h wird bei R.T. gerührt, das Solvens im Vakuum entfernt, in 100 ml MTB aufgenommen und mit je 100 ml $NaHSO_4$-Lösung und $NaHCO_3$-Lösung gewaschen. Über $Na_2SO_4$ wird getrocknet, das Solvens im Vakuum entfernt und mit EE/H 1:1 chromatographiert. Man erhält 2 diastereomere Öle, die getrennt weiter verarbeitet werden.

| | |
|---|---|
| Diastereomer 1 $R_f$ (EE/H 1:1) = 0,30 | 0,95 g |
| Diastereomer 2 $R_f$ (EE/H 1:1) = 0,20 | 0,95 g |

MS (DCI, für beide Diastereomere gleich): 404 (M+1)

c) [3-t-Butylsulfonyl,2-(2-thienylmethyl)]propionsäure

4,4 g [3-t-Butylsulfonyl,2-(2-thienylmethyl)]propionsäure-methylester werden in 50 ml 5N HCl suspendiert und 2 h zum Rückfluß erhitzt. Nach Abkühlung wird 3 x mit 50 ml EE extrahiert, über $Na_2SO_4$ getrocknet und das Solvens im Vakuum entfernt. Man erhält 4,0 g der Titelverbindung als blaßgelbes Öl.

$R_f$ (MTB) = 0,15 - 0,25  MS (DCI): 291 (M+1)

d) [3-t-Butylsulfonyl,2-(2-thienylmethyl)]propionsäure-methylester

8,4 g [3-t-Butylthio,2-(2-thienylmethyl)]propionsäure-methylester werden in 100 ml $CH_2Cl_2$ gelöst und 10,6 g m-Chlorperbenzoesäure unter Eiskühlung portionsweise zugegeben. 1 h wird bei R.T. gerührt, anschließend zunächst mit 100 ml 10 % $Na_2SO_3$, dann mit 100 ml $NaHCO_3$ gewaschen. Über $Na_2SO_4$ wird getrocknet und das Solvens im Vakuum entfernt. Man erhält 7,2 g der Titelverbindung als farbloses Öl.

$R_f$ (MTB) = 0,56  MS (DCI): 305 (M+1)

e) [3-t-Butylthio,2-(2-thienylmethyl)]propionsäure-methylester

6,1 ml t-Butylmercaptan werden in 100 ml MeOH (wasserfrei) gelöst und unter Argon 130 mg NaH zugegeben. Anschließend werden 7,6 g 2-(2-Thienylmethyl)acrylsäure-methylester zugetropft und 4 h bei R.T. gerührt. Das Solvens wird im Vakuum entfernt, in 100 ml MTB aufgenommen und mit 100 ml 5 % $NaHSO_4$-Lösung gewaschen. Über $Na_2SO_4$ wird getrocknet und das Solvens im Vakuum entfernt. Man erhält 10,4 g der Titelverbindung als blaßgelbe Flüssigkeit, die ohne Reinigung und Charakterisierung weiter eingesetzt wird.

$R_f$ (DIP/H 1:5) = 0,31

f) 2-(2-Thienylmethyl)acrylsäure-methylester

11,8 g 2-Thienylmethylmalonsäure-monomethylester, 5,8 ml Diethylamin und 5,0 ml 36 % wäßrige Formaldehyd-Lösung werden bei R.T. unter Argon 1 h gerührt. Das Wasser wird anschließend im Vakuum entfernt und der Rückstand chromatographiert. Man erhält 7,6 g der Titelverbindung als farblose Flüssigkeit.

$R_f$ (MTB/H 1:5) = 0,49

g) 2-Thienylmethylmalonsäure-monomethylester

20,1 g 2-Thienylmethylmalonsäure-dimethylester werden in 300 ml MeOH gelöst und 5,0 g KOH zugegeben. Bei R.T. wird 7 h gerührt, das Wasser im Vakuum entfernt und mit je 100 ml 5 % $Na_2CO_3$-Lösung und EE aufgenommen. Anschließend wird die wäßrige Phase auf pH=2 angesäuert und 3 x mit je 100 ml EE extrahiert. Über $Na_2SO_4$ wird getrocknet und das Solvens im Vakuum entfernt. Man erhält 16,0 g der Titelverbindung als blaßgelbes Öl.

$R_f$ (EE/MeOH 6:1) = 0,3 - 0,4

h) 2-Thienylmethylmalonsäure-dimethylester

87,8 g Malonsäuredimethylester und 41,0 g Kalium-t-butylat werden unter Eiskühlung in 1,1 l THF (wasserfrei) gelöst und unter Argon 44,1 g 2-Thienylmethylchlorid in 500 ml THF zugetropft. 3 h wird bei R.T. gerührt, das KCl abfiltriert, das Solvens im Vakuum entfernt und der Rückstand chromatographiert. Man erhält 33,8 g der Titelverbindung (I) als farbloses Öl neben 8,8 g Bis-(2-Thienylmethyl)malonsäuredimethylester (II)

$R_f$ (I) (Toluol/DIP 20:1) = 0,35

$R_f$ (II) (Toluol/DIP 20:1) = 0,44

g) 2-Thienylmethylchlorid

252 g Thiophen werden in 128 ml konzentrierter wäßriger HCl suspendiert und bei 0°C 1 h lang HCl-Gas eingeleitet. Anschließend werden ohne Unterbrechung des HCl-Stromes 255 ml 35 % wäßrige Formaldehydlösung zugetropft und weitere 15 Minuten bei 0°C gerührt. Nach Abtrennung der organischen Phase wird die wäßrige Phase noch 2 x mit 600 ml $CH_2Cl_2$ extrahiert. Anschließend wird 2 x mit 600 ml gesättigter wäßriger $Na_2CO_3$ gewaschen, über $Na_2SO_4$ getrocknet, das Solvens im Vakuum entfernt und destilliert. Man erhält 174 g der Titelverbindung als farblose Flüssigkeit.

$Sdp_{22}$ = 81 - 84°C

Die Verbindungen der Beispiele 36-44 werden analog Beispiel 35 hergestellt.

**Beispiel 36**

(2-Benzyl,3-t-butylsulfonyl)propionyl-Nva-[Cyclohexylmethyl,(4,4,5,5-tetramethyl-1,3,2-dioxaborolan-2-yl)]methylamid

$R_f$ (MTB) = 0,38 MS (FAB): 619 (M+1)

**Beispiel 37**

(3-t-Butylsulfonyl)propionyl-Nva-[Cycloundecylmethyl, (4,4,5,5-tetramethyl-1,3,2-dioxaborolan-2-yl)]methylamid

$R_f$ (EE) = 0,24 MS (FAB): 599 (M+1)

**Beispiel 38**

(3-t-Butylsulfonyl)propionyl-Nva-[1-Naphthylmethyl, (4,4,5,5-tetramethyl-1,3,2-dioxaborolan-2-yl)]methylamid

$R_f$ (EE) = 0,23 MS (FAB): 573 (M+1)

**Beispiel 39**

(2-Benzyl,3-t-butylsulfonyl)propionyl-Val-[Cyclohexylmethyl,(4,4,5,5-tetramethyl-1,3,2-dioxaborolan-2-yl)]methylamid

$R_f$ (MTB) = 0,25 MS (FAB): 619 (M+1)

**Beispiel 40**

[3-t-Butylsulfonyl,2-(2-thienylmethyl)]propionyl-Thi-[Cycloheptylmethyl,(4,4,5,5-tetramethyl-1,3,2-dioxaborolan-2-yl)]methylamid

$R_f$ (EE/H 1:1) = 0,23 MS (FAB): 693 (M+1)

**Beispiel 41**

[3-t-Butylsulfonyl,2-(2-thienylmethyl)]propionyl-Nva-[Cycloheptylmethyl,(4,4,5,5-tetramethyl-1,3,2-dioxaborolan-2-yl)]methylamid

$R_f$ (MTB) = 0,35 MS (FAB): 639 (M+1)

**Beispiel 42**

[3-t-Butylsulfonyl,2-(1-naphthylmethyl)]propionyl-Nva-[3-methyl,1-(4,4,5,5-tetramethyl-1,3,2-dioxaborolan-2-yl)]butylamid

$R_f$ (MTB) = 0,26 MS (FAB): 629 (M+1)

**Beispiel 43**

[3-t-Butylsulfonyl,2-(2-thienylmethyl)]propionyl-Thi-[Cyclohexylmethyl,(4,4,5,5-tetramethyl-1,3,2-dioxaborolan-2-yl)]methylamid
$R_f$ (MTB) = 0,53 MS (FAB): 679 (M+1)

**Beispiel 44**

(2-Benzyl,3-t-butylsulfonyl)propionyl-Nva-[Cyclopentylmethyl,(4,4,5,5-tetramethyl-1,3,2-dioxaborolan-2-yl)]methylamid
$R_f$ (MTB) = 0,36 MS (FAB): 605 (M+1)

**Beispiel 45**

[3-t-Butylsulfonyl,2-(2-methyl)benzyl]propionyl-Nva-[Cyclohexylmethyl,(4,4,5,5-tetramethyl-1,3,2-dioxaborolan-2-yl)]methylamid
$R_f$ (MTB) = 0,40 MS (FAB): 633 (M+1)

**Beispiel 46**

[3-t-Butylsulfonyl,2-(3-methyl)benzyl]propionyl-Nva-[Cyclohexylmethyl,(4,4,5,5-tetramethyl-1,3,2-dioxaborolan-2-yl)]methylamid
$R_f$ (MTB) = 0,40 MS (FAB): 633 (M+1)

**Beispiel 47**

(2-Benzyl,3-t-butylsulfonyl)propionyl-Nva-[Benzyl,(4,4,5,5-tetramethyl-1,3,2-dioxaborolan-2-yl)]methylamid
$R_f$ (MTB) = 0,30 MS (FAB): 613 (M+1)

**Beispiel 48**

(2-Benzyl,3-t-butylsulfonyl)propionyl-Asn-[Benzyl,(4,4,5,5-tetramethyl-1,3,2-dioxaborolan-2-yl)]methylamid
$R_f$ (EE) = 0,05 MS (FAB): 628 (M+1)

**Beispiel 49**

(2-Benzyl,3-t-butylsulfonyl)propionyl-Nva-[Cycloheptyl,-(4,4,5,5-tetramethyl-1,3,2-dioxaborolan-2-yl)]methylamid
$R_f$ (MTB) = 0,30 MS (FAB): 619 (M+1)

**Beispiel 50**

[3-t-Butylsulfonyl,2-(1-naphthylmethyl)]propionyl-Nva-[Cyclopentylmethyl,(4,4,5,5-tetramethyl-1,3,2-dioxaborolan-2-yl]-methylamid
$R_f$ (MTB) = 0,40 MS (FAB): 655 (M+1)

**Beispiel 51**

[3-t-Butylsulfonyl,2-(1-naphthylmethyl)]propionyl-Nva-[3-Ethyl,1-(4,4,5,5-tetramethyl-1,3,2-dioxaborolan-2-yl)]pentylamid
$R_f$ (MTB) = 0,50 MS (FAB): 657 (M+1)

**Beispiel 52**

(2-Benzyl,3-t-butylsulfonyl)propionyl-Nva-[3-Ethyl,1-(4,4,5,5-tetramethyl-1,3,2-dioxaborolan-2-yl)]pentylamid
$R_f$ (MTB) = 0,44 MS (FAB): 607 (M+1)

**Beispiel 53**

(2-Benzyl,3-t-butylsulfonyl)propionyl-Nva-[3-methyl,1-(4,4,5,5-tetramethyl-1,3,2-dioxaborolan-2-yl)]butylamid
$R_f$ (MTB) = 0,35 MS (FAB): 579 (M+1)

**Beispiel 54**

(3-t-Butylsulfonyl,2-(2-thienylmethyl)]propionyl-Asn-[Cyclohexylmethyl,(4,4,5,5-tetramethyl-1,3,2-dioxaborolan-2-yl)]methylamid
$R_f$ (EE/Aceton 5:1) = 0,30    MS (FAB): 640 (M+1)

## Patentansprüche

1. Verbindung der Formel I

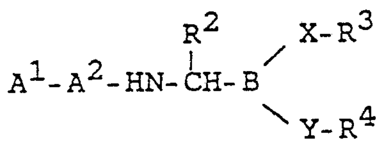

(I)

in welcher
$A^1$ einen Rest der Formeln II, III, IV, V oder VI bedeutet

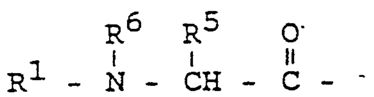

(II)

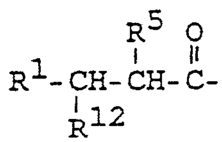

(III)

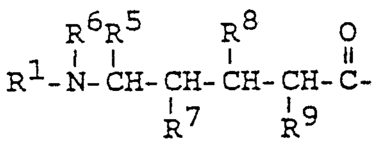

(IV)

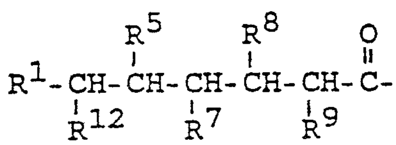

(V)

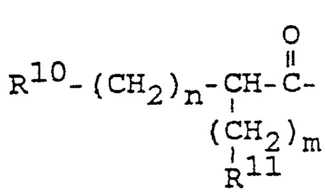

(VI)

worin
$R^1$ a₁) Wasserstoff, $(C_1-C_{12})$-Alkyl, das gegebenenfalls ein-oder zweifach ungesättigt ist und das gegebenenfalls durch bis zu 3 gleiche oder verschiedene Reste aus der Reihe Hydroxy, $(C_1-C_7)$-Alkoxy, Carbamoyl, $(C_1-C_8)$-Alkanoyloxy, Carboxy, $(C_1-C_7)$-Alkoxycarbonyl, F, Cl, Br, I, Amino, Amidino, das

gegebenenfalls durch einen, zwei oder drei ($C_1$-$C_8$)-Alkylreste substituiert sein kann, Guanidino, das gegebenenfalls durch einen, zwei, drei oder vier ($C_1$-$C_8$)-Alkylreste substituiert sein kann, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethoxycarbonylamino substituiert ist;
mono-, bi- oder tricyclisches ($C_3$-$C_{18}$)-Cycloalkyl, ($C_3$-$C_{18}$)-Cycloalkyl-($C_1$-$C_6$)-alkyl oder ($C_6$-$C_{14}$)-Aryl, wobei der Cycloalkylteil gegebenenfalls durch ($C_1$-$C_6$)-Alkyl substituiert ist und Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogenen substituiertes Anilino und Trifluormethyl substituiert ist;
($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist;
oder für den Rest eines 5- oder 6-gliedrigen monocyclischen oder 9-oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht, und der gegebenenfalls wie ($C_6$-$C_{14}$)-Aryl unter $a_1$) definiert, mono-, di- oder trisubstituiert ist, bedeutet
oder
$a_2$) einen Rest der Formel VII bedeutet

$R^{1'}$ - W (VII)

worin $R^{1'}$ wie $R^1$ unter $a_1$) definiert ist und W für -CO-, -O-CO-, $-SO_2-$, -SO-, $-HN-SO_2-$, -HN-CO-, -CH(OH)- oder -N(OH)- steht,
$R^2$, $R^5$ und $R^9$ unabhängig voneinander wie $R^1$ unter $a_1$) definiert sind,
$R^3$ und $R^4$ unabhängig voneinander Wasserstoff oder ($C_1$-$C_{12}$)-Alkyl bedeuten, wobei Aryl gegebenenfalls ein bis zweifach ungesättigt ist und gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist; oder zusammen mit Bor, X und Y ein mono-, bi- oder tricyclisches, gesättigtes oder teilweise ungesättigtes mono-, di-, tri- oder tetra-($C_1$-$C_{12}$)-alkyliertes Ringsystem mit 5-18 Ringgliedern, das außer Bor, X, Y und Kohlenstoff noch ein -O- Glied, ein $-NR^{13}$-Glied oder ein $-CR^{14}R^{15}$-Glied enthalten kann, bilden,
X und Y unabhängig voneinander für -O- oder $-NR^{13}$- stehen,
$R^6$ für Wasserstoff oder ($C_1$-$C_8$)-Alkyl steht, oder zusammen mit $R^5$ ein mono- oder bicyclisches, gesättigtes oder teilweise ungesättigtes Ringsystem mit 5-12 Ringgliedern bildet,
$R^7$ und $R^8$ unabhängig voneinander Wasserstoff; Hydroxy; Amino; Fluor; Amino-($C_1$-$C_4$)-alkyl; Hydroxy-($C_1$-$C_4$)-alkyl; ($C_1$-$C_4$)-Alkyl, das auch einfach ungesättigt sein kann, bedeuten,
$R^{10}$ und $R^{11}$ unabhängig voneinander Wasserstoff, Hydroxy oder ($C_6$-$C_{14}$)-Aryl bedeuten, wobei Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5-oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atom und/oder 1 - 2 S-Atomen und/oder 1 - 2 O-Atomen als Ringglieder stehen, der gegebenenfalls wie oben ($C_6$-$C_{14}$)-Aryl mono- oder disubstituiert ist,
n und m unabhängig voneinander 0, 1, 2, 3 und 4 sein können,
$R^{12}$ Wasserstoff oder ($C_1$-$C_8$)-Alkyl ist, oder gemeinsam mit $R^1$ ein mono- oder bicyclisches, gesättigtes oder teilweise ungesättigtes Ringsystem mit 5 - 12 Ringgliedern bildet, das außer Kohlenstoff noch 1 Schwefelatom enthalten kann, welches gegebenenfalls zum Sulfoxid oder Sulfon oxidiert sein kann,
$A^2$ entweder abwesend ist, wobei gleichzeitig $A^1$ nicht für einen Rest der Formel II steht, oder einen N-terminal mit $A^1$ und C-terminal mit dem Aminoboronsäurederivat verknüpften Rest der Formel VIII bedeutet,

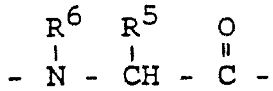

(VIII)

wobei $R^5$ und $R^6$ wie oben definiert sind,
$R^{13}$ Wasserstoff oder ($C_1$-$C_{12}$)-Alkyl, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, bedeutet,

$R^{14}$ und $R^{15}$ unabhängig voneinander Wasserstoff, ($C_1$-$C_8$)-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)amino, Bis(2-hydroxyethyl)amino bedeuten sowie deren physiologisch verträgliche Salze, wobei die Verbindungen Boc-Phe-Pro-[Benzyl,(4,4,5,5-tetramethyl-1,3,2-dioxaborolan-2-yl)]methylamid, Boc-Phe-Gly-[Isobutyl,(4,4,5,5-tetramethyl-1,3,2-dioxaborolan-2-yl)]methylamid, Boc-Phe-Gly-[Isobutyl,[(N-B)-(2,2′-iminodiethanolato) boryl]]methylamid, H-Phe-Gly-[Isobutyl,(4,4,5,5-tetramethyl-1,3,2-dioxaborolan-2-yl)]methylamid•Trifluoracetat und Boc-D-Phe-Pro-[Isopropyl,(4,4,5,5-tetramethyl-1,3,2-dioxaborolan-2-yl)]methylamid ausgenommen sind.

2. Verbindung der Formel I gemäß Anspruch 1, in welcher
$A^1$ ist wie in Anspruch 1 definiert ist,
$R^1$ vorzugsweise Wasserstoff bedeutet oder für ($C_1$-$C_{10}$)-Alkyl; Cyclopentyl; Cyclohexyl; Cyclopentyl-($C_1$-$C_{10}$)-alkyl; Cyclohexyl-($C_1$-$C_{10}$)-alkyl; gegebenenfalls substituiertes Phenyl-($C_1$-$C_8$)-alkyl; 2-Pyridyl-($C_1$-$C_8$)-alkyl; 3-Pyridyl-($C_1$-$C_8$)-alkyl; 4-Pyridyl-($C_1$-$C_8$)-alkyl; $H_2N$-($C_1$-$C_{10}$)-Alkyl; HO-($C_1$-$C_{10}$)-Alkyl; ($C_1$-$C_4$)-Alkoxy-($C_1$-$C_{10}$)-alkyl; ($C_1$-$C_4$)-Alkoxycarbonyl-($C_1$-$C_{10}$)-alkyl; ($C_1$-$C_8$)-Alkylsulfonyl; ($C_1$-$C_8$)-Alkylsulfinyl; Hydroxy-($C_1$-$C_{10}$)-alkanoyl; ($C_1$-$C_8$)-Alkanoyloxy-($C_1$-$C_{10}$)-alkyl; ($C_1$-$C_{11}$)-Alkanoyl; gegebenenfalls geschütztes Amino-($C_1$-$C_{11}$)-alkanoyl; Di-($C_1$-$C_7$)-alkylamino-($C_2$-$C_{11}$)-alkanoyl; ($C_3$-$C_9$)-Cycloalkylcarbonyl; ($C_6$-$C_{10}$)-Aryl-($C_2$-$C_{11}$)-alkanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl; Pyrrolyl-2-carbonyl; Pyridyl-3-carbonyl; Benzolsulfonyl; ($C_1$-$C_{10}$)-Alkoxycarbonyl; substituiertes ($C_1$-$C_{10}$)-Alkoxycarbonyl; ($C_6$-$C_{14}$)-Aryl-($C_1$-$C_6$)-alkoxycarbonyl steht; oder $R^1$ gemeinsam mit $R^{12}$ ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxydiert sein kann,
$R^2$ ($C_3$-$C_{12}$)-Alkyl; mono-, bi- oder tricyclisches ($C_3$-$C_{18}$)-Cycloalkyl, ($C_3$-$C_{18}$)-Cycloalkylmethyl, ($C_3$-$C_{18}$)-Cycloalkylethyl, wobei der Cycloalkylteil gegebenenfalls durch ($C_1$-$C_6$)-Alkyl substituiert ist; Dithiolanyl; ($C_6$-$C_{14}$)-Arylmethyl; Dithiolanylmethyl; Dithiolanylethyl; Dithianyl; Dithianylmethyl oder Dithianylethyl ist,
$R^3$ und $R^4$ unabhängig voneinander Wasserstoff oder ($C_1$-$C_{12}$)-Alkyl sind oder zusammen mit Bor, X und Y ein mono-, bi-oder tricyclisches, gesättigtes oder teilweise ungesättigtes, gegebenenfalls mono-, di-, tri- oder tetra-($C_1$-$C_{12}$)-alkyliertes Ringsystem mit 5-18 Ringgliedern bilden, das außer Bor, X, Y und Kohlenstoff noch ein -O- Glied, ein -$NR^{13}$-Glied oder ein -$CR^{14}R^{15}$-Glied enthalten kann,
X und Y unabhängig voneinander -O- oder -$NR^{13}$- sind,
$R^5$ und $R^9$ unabhängig voneinander Wasserstoff; ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)Alkoxy, ($C_1$-$C_7$)-Alkanoyloxy, Carboxy, ($C_1$-$C_7$)Alkoxycarbonyl, Cl, Br, Amino, Amidino, Guanidino, Carbamoyl, ($C_1$-$C_5$)-Alkoxycarbonylamino, ($C_7$-$C_{15}$)-Aralkoxycarbonylamino und 9-Fluorenylmethoxycarbonylamino substituiert ist; ($C_3$-$C_{12}$)-Cycloalkyl-($C_1$-$C_3$)-alkyl, mono- oder bicyclisches ($C_6$-$C_{14}$)-Aryl-($C_1$-$C_3$)-Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist, bedeuten; oder für ($C_1$-$C_3$)-Alkyl, substituiert mit dem Rest eines 5- oder 6-gliedrigen monocyclischen oder 9- oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie in Anspruch 1 für ($C_6$-$C_{14}$)-Aryl beschrieben mono- oder disubstituiert ist, stehen,
$R^6$ Wasserstoff, Methyl oder Ethyl ist oder zusammen mit $R^5$ Pyrrolidin oder Piperidin, die jeweils noch mit Cyclopentyl, Cyclohexyl oder Phenyl anneliert sein können, bildet,
$R^7$ und $R^8$ unabhängig voneinander Wasserstoff, Hydroxy, Amino, Fluor, Hydroxymethyl oder Aminomethyl sind,
$R^{10}$ und $R^{11}$ unabhängig voneinander Wasserstoff, Hydroxy, Phenyl, 2- oder 3-Thienyl, 2-, 3- oder 4-Pyridyl, 1-, 2- oder 4-Imidazolyl, 1- oder 2-Naphthyl; 2- oder 3-Benzo[b]thienyl sind,
n und m unabhängig voneinander 0, 1, 2, 3 und 4 bedeuten können,
$R^{12}$ wie in Anspruch 1 definiert ist,
$R^{13}$ Wasserstoff oder ($C_1$-$C_{12}$)-Alkyl ist,
$R^{14}$ und $R^{15}$ wie in Anspruch 1 definiert sind,
$A^2$ abwesend ist, wobei gleichzeitig $A^1$ nicht für einen Rest der Formel II steht, oder einen Rest der Formel VIII bedeutet, wobei $R^5$ und $R^6$ wie vorstehend beschrieben definiert sind,
sowie deren physiologisch verträgliche Salze.

3. Verbindung der Formel I, gemäß einem oder mehreren der Ansprüche 1 bis 2, in welcher
$A^1$ wie in Anspruch 1 definiert ist,
$R^1$ ($C_1$-$C_8$)-Alkylsulfonyl; ($C_1$-$C_8$)-Alkylsulfinyl; Hydroxy-($C_1$-$C_{10}$)-alkanoyl; ($C_1$-$C_8$)-Alkanoyloxy-($C_1$-$C_{10}$)-alkyl; ($C_1$-$C_{11}$)-Alkanoyl; Amino-($C_1$-$C_{11}$)-alkanoyl; Di-($C_1$-$C_7$)- alkylamino-($C_2$-$C_{11}$)-alkanoyl; ($C_3$-$C_9$)-Cycloalkylcarbonyl; ($C_6$-$C_{10}$)-Aryl-($C_2$-$C_{11}$)-alkanoyl; 2-Pyridyl-($C_1$-$C_8$)-alkanoyl; 3-Pyridyl-($C_1$-$C_8$)-alkanoyl; 4-Pyridyl-($C_1$-$C_8$)-alkanoyl; gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl; Pyrrolyl-2-carbonyl; Pyridyl-3-carbonyl; Benzolsulfonyl; ($C_1$-$C_{10}$)-Alkoxycarbonyl; substituiertes ($C_1$-$C_{10}$)-Alkoxycarbonyl; ($C_6$-$C_{14}$)-Aryl-

$(C_1-C_6)$-alkoxycarbonyl bedeutet,
$R^2$ $(C_3-C_{12})$-Alkyl; mono-, bi- oder tricyclisches $(C_3-C_{18})$-Cycloalkyl oder $(C_3-C_{18})$-Cycloalkylmethyl, wobei der Cycloalkylteil gegebenenfalls durch $(C_1-C_4)$-Alkyl substituiert ist; $(C_6-C_{14})$-Arylmethyl; Dithiolanyl; Dithiolanylmethyl; Dithianyl und Dithianylmethyl bedeutet,
$R^3$ und $R^4$ Wasserstoff sind oder gemeinsam mit Bor, X und Y ein mono-, bi- oder tricyclisches mono-, di-, tri- oder tetra-$(C_1-C_{12})$-alkyliertes, gesättigtes oder teilweise ungesättigtes Ringsystem mit 5-18 Ringgliedern bilden, das außer Bor, X, Y und Kohlenstoff noch ein -O- Glied, ein -$NR^{13}$-Glied oder ein -$CR^{14}R^{15}$-Glied enthalten kann,
X und Y für -O- stehen,
$R^5$ und $R^9$ unabhängig voneinander Wasserstoff, Methyl, Ethyl, Isopropyl, n-Propyl, N-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto,1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)ethyl, 3-Thienyl, 3-Thienylmethyl, 2-(3Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methyl-imidazol-4-yl)methyl, (3-Methyl-imidazol-4-yl)methyl, Phenyl, 1-Naphthylmethyl, 2-Naphthylmethyl, 2-Phenylmethyl, 2-Thiazolylmethyl, 4-Thiazolylmethyl, 3-Pyrazolylmethyl, 4-Pyrimidinylmethyl, Indol-2-yl-methyl, 2-Benzo[b]thienylmethyl, 3-Benzo[b]thienylmethyl, 2-Furylmethyl sind.
$R^6$ Wasserstoff oder Methyl bedeuten oder gemeinsam mit $R^5$ und der diese Reste tragenden -N-CH-Gruppe ein Tetrahydroisochinolin- oder Azabicyclooctan-Gerüst bildet,
einer der Reste $R^7$ oder $R^8$ Wasserstoff bedeutet, der jeweils andere Rest Hydroxy, Amino, Fluor, Hydroxymethyl oder Aminomethyl,
$R^{10}$ und $R^{11}$ unabhängig voneinander Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl sind,
n und m unabhängig voneinander 0, 1 oder 2 bedeuten,
$R^{12}$ Wasserstoff oder Methyl ist, oder gemeinsam mit $R^1$ ein mono- oder bicyclisches gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann,
$R^{13}$ Wasserstoff oder $(C_1-C_4)$-Alkyl ist,
$R^{14}$ und $R^{15}$ wie in Anspruch 1 definiert sind,
$A^2$ abwesend ist, wobei gleichzeitig $A^1$ nicht für einen Rest der Formel II steht, oder einen Rest der Formel VIII bedeutet, wobei $R^5$ und $R^6$ wie in Anspruch 1 definiert sind,
sowie deren physiologisch verträgliche Salze.

4. Verbindung der Formel I, gemäß einem oder mehreren der Ansprüche 1 bis 3, in welcher
$A^1$ für einen Rest der Formel II, III oder VI steht, worin
$R^1$ $(C_1-C_6)$-Alkylsulfonyl; $(C_1-C_6)$-Alkylsulfinyl; Amino-$(C_1-C_6)$-alkanoyl; $(C_1-C_6)$-Alkoxycarbonyl; $(C_6-C_{10})$-Aryl-$(C_1-C_6)$-alkoxycarbonyl bedeutet,
$R^2$ $(C_5-C_8)$-Cycloalkyl; $(C_5-C_{11})$-Cycloalkylmethyl; [$(C_1-C_4)$-Alkyl-cyclohexyl]methyl; $(C_3-C_4)$-Alkyl, das durch $(C_1-C_3)$-Alkyl substituiert ist; $(C_6-C_{10})$-Arylmethyl oder Dithiolan-2-yl-methyl bedeutet,
$R^3$ und $R^4$ für Wasserstoff stehen oder zusammen mit Bor, X und Y einen 1,3,2-Dioxaborolan-Rest, der gegebenenfalls durch 1 bis 4 Methylgruppen substituiert ist, einen [4,5-Diisopropyl]-1,3,2-dioxaborolan-, einen [(N-B)-(2,2′-imino-diethanolato)-boryl)]-, einen Pinandioxyboryl- oder einen 1,3,2-Dioxaborinan-Rest, dessen C-Atom in Position 5 durch $R^{14}$ und $R^{15}$ substituiert ist, bilden,
X und Y für -O- stehen,
$R^5$ Wasserstoff, n-Propyl, Isopropyl, n-Butyl, Isobutyl, sec.-Butyl, Carbamoylmethyl, 2-Carbamoylethyl, Benzyl, 2-Methylbenzyl, 3-Methylbenzyl, 2-Thienylmethyl, 3-Thienylmethyl, 1-Naphthylmethyl, 2-Naphthylmethyl, Imidazol-4-yl-methyl oder 2-Thiazolylmethyl bedeutet,
$R^6$ für Wasserstoff oder Methyl steht,
$R^{10}$ und $R^{11}$ unabhängig voneinander 1-Naphthyl oder 2-Thienyl bedeuten,
n und m für 1 stehen,
$R^{12}$ Wasserstoff bedeutet,
$R^{14}$ und $R^{15}$ unabhängig voneinander Wasserstoff, $(C_1-C_8)$-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)amino oder Bis(2-hydroxyethyl)amino bedeuten,
und
$A^2$ für einen Rest der Formel VIII steht, wobei $R^5$ und $R^6$ wie in Anspruch 3 definiert sind,
sowie deren physiologisch verträgliche Salze.

5. Verfahren zur Herstellung von Verbindungen der Formel I, gemäß einem oder mehreren der Ansprüche 1 bis 4, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

6. Verbindung der Formel I gemäß einem oder mehreren der Ansprüche 1 bis 4 zur Anwendung als Heilmittel.

7. Verbindung der Formel I gemäß einem oder mehreren der Ansprüche 1 bis 4 zur Anwendung als Heilmittel bei der Behandlung des Bluthochdrucks.

8. Pharmazeutisches Mittel enthaltend eine Verbindung der Formel I gemäß einem oder mehreren der Ansprüche 1 bis 4.

**Patentansprüche für folgende Vertragsstaaten: ES, GR**

1. Verfahren zur Herstellung einer Verbindung der Formel I

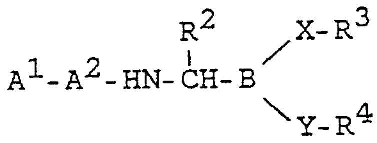

(I)

in welcher

$A^1$ einen Rest der Formeln II, III, IV, V oder VI bedeutet

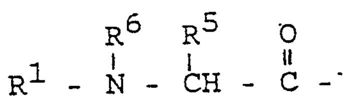

(II)

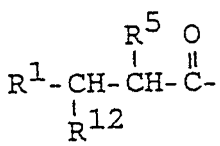

(III)

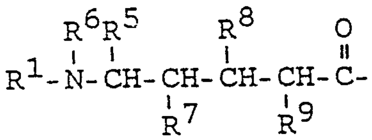

(IV)

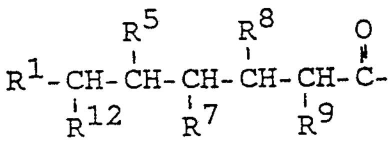

(V)

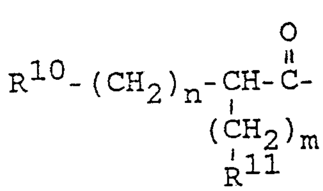

(VI)

worin

$R^1$ $a_1$) Wasserstoff, $(C_1-C_{12})$-Alkyl, das gegebenenfalls ein-oder zweifach ungesättigt ist und das gegebenenfalls durch bis zu 3 gleiche oder verschiedene Reste aus der Reihe Hydroxy, $(C_1-C_7)$-Alkoxy, Carbamoyl, $(C_1-C_8)$-Alkanoyloxy, Carboxy, $(C_1-C_7)$-Alkoxycarbonyl, F, Cl, Br, I, Amino, Amidino, das gegebenenfalls durch einen, zwei oder drei $(C_1-C_8)$-Alkylreste substituiert sein kann, Guanidino, das

gegebenenfalls durch einen, zwei, drei oder vier ($C_1$-$C_8$)-Alkylreste substituiert sein kann, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethoxycarbonylamino substituiert ist;

mono-, bi- oder tricyclisches ($C_3$-$C_{18}$)-Cycloalkyl, ($C_3$-$C_{18}$)-Cycloalkyl-($C_1$-$C_6$)-alkyl oder ($C_6$-$C_{14}$)-Aryl, wobei der Cycloalkylteil gegebenenfalls durch ($C_1$-$C_6$)-Alkyl substituiert ist und Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogenen substituiertes Anilino und Trifluormethyl substituiert ist;

($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist;

oder für den Rest eines 5- oder 6-gliedrigen monocyclischen oder 9-oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht, und der gegebenenfalls wie ($C_6$-$C_{14}$)-Aryl unter $a_1$) definiert, mono-, di- oder trisubstituiert ist, bedeutet

oder

$a_2$) einen Rest der Formel VII bedeutet

$R^{1'}$ - W (VII)

worin $R^{1'}$ wie $R^1$ unter $a_1$) definiert ist und W für -CO-, -O-CO-, -$SO_2$-, -SO-, -HN-$SO_2$-, -HN-CO-, -CH(OH)- oder -N(OH)- steht,

$R^2$, $R^5$ und $R^9$ unabhängig voneinander wie $R^1$ unter $a_1$) definiert sind,

$R^3$ und $R^4$ unabhängig voneinander Wasserstoff oder ($C_1$-$C_{12}$)-Alkyl bedeuten, wobei Aryl gegebenenfalls ein-bis zweifach ungesättigt ist und gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist; oder zusammen mit Bor, X und Y ein mono-, bi- oder tricyclisches, gesättigtes oder teilweise ungesättigtes mono-, di-, tri- oder tetra-($C_1$-$C_{12}$)-alkyliertes Ringsystem mit 5-18 Ringgliedern, das außer Bor, X, Y und Kohlenstoff noch ein -O- Glied, ein -$NR^{13}$-Glied oder ein -$CR^{14}R^{15}$-Glied enthalten kann, bilden,

X und Y unabhängig voneinander für -O- oder -$NR^{13}$- stehen,

$R^6$ für Wasserstoff oder ($C_1$-$C_8$)-Alkyl steht, oder zusammen mit $R^5$ ein mono- oder bicyclisches, gesättigtes oder teilweise ungesättigtes Ringsystem mit 5-12 Ringgliedern bildet,

$R^7$ und $R^8$ unabhängig voneinander Wasserstoff; Hydroxy; Amino; Fluor; Amino-($C_1$-$C_4$)-alkyl; Hydroxy-($C_1$-$C_4$)-alkyl; ($C_1$-$C_4$)-Alkyl, das auch einfach ungesättigt sein kann, bedeuten,

$R^{10}$ und $R^{11}$ unabhängig voneinander Wasserstoff, Hydroxy oder ($C_6$-$C_{14}$)-Aryl bedeuten, wobei Aryl gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist; oder für den Rest eines 5-oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atom und/oder 1 - 2 S-Atomen und/oder 1 - 2 O-Atomen als Ringglieder stehen, der gegebenenfalls wie oben ($C_6$-$C_{14}$)-Aryl mono- oder disubstituiert ist,

n und m unabhängig voneinander 0, 1, 2, 3 und 4 sein können,

$R^{12}$ Wasserstoff oder ($C_1$-$C_8$)-Alkyl ist, oder gemeinsam mit $R^1$ ein mono- oder bicyclisches, gesättigtes oder teilweise ungesättigtes Ringsystem mit 5 - 12 Ringgliedern bildet, das außer Kohlenstoff noch 1 Schwefelatom enthalten kann, welches gegebenenfalls zum Sulfoxid oder Sulfon oxidiert sein kann,

$A^2$ entweder abwesend ist, wobei gleichzeitig $A^1$ nicht für einen Rest der Formel II steht, oder einen N-terminal mit $A^1$ und C-terminal mit dem Aminoboronsäurederivat verknüpften Rest der Formel VIII bedeutet,

```
  R6   R5    O
  |    |     ||
- N  - CH  - C  -          (VIII)
```

wobei $R^5$ und $R^6$ wie oben definiert sind,

$R^{13}$ Wasserstoff oder ($C_1$-$C_{12}$)-Alkyl, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, bedeutet,

$R^{14}$ und $R^{15}$ unabhängig voneinander Wasserstoff, ($C_1$-$C_8$)-Alkyl, Hydroxymethyl, 2-Hydroxyethyl,

(3-Hydroxysulfonyl, 2-hydroxypropyl)amino, (2-Hydroxysulfonylethyl)amino, (2-Hydroxysulfonylpropyl)amino, (Carboxymethyl)amino, Bis(2-hydroxyethyl)amino bedeuten sowie deren physiologisch verträgliche Salze, wobei die Verbindungen Boc-Phe-Pro-[Benzyl,(4,4,5,5-tetramethyl-1,3,2-dioxaborolan-2-yl)]methylamid, Boc-Phe-Gly-[Isobutyl,(4,4,5,5-tetramethyl-1,3,2-dioxaborolan-2-yl)]methylamid, Boc-Phe-Gly-[Isobutyl,[(N-B)-(2,2′-iminodiethanolato) boryl]]methylamid, H-Phe-Gly-[Isobutyl,(4,4,5,5-tetramethyl-1,3,2-dioxaborolan-2-yl)]methylamid•Trifluoracetat und Boc-D-Phe-Pro-[Isopropyl,(4,4,5,5-tetramethyl-1,3,2-dioxaborolan-2-yl)]methylamid ausgenommen sind, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktionelle Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß man eine Verbindung der Formel I herstellt, in welcher
$A^1$ ist wie in Anspruch 1 definiert ist,
$R^1$ vorzugsweise Wasserstoff bedeutet oder für $(C_1-C_{10})$-Alkyl; Cyclopentyl; Cyclohexyl; Cyclopentyl-$(C_1-C_{10})$-alkyl; Cyclohexyl-$(C_1-C_{10})$-alkyl; gegebenenfalls substituiertes Phenyl-$(C_1-C_8)$-alkyl; 2-Pyridyl-$(C_1-C_8)$-alkyl; 3-Pyridyl-$(C_1-C_8)$-alkyl; 4-Pyridyl-$(C_1-C_8)$-alkyl; $H_2N$-$(C_1-C_{10})$-Alkyl; HO-$(C_1-C_{10})$-Alkyl; $(C_1-C_4)$-Alkoxy-$(C_1-C_{10})$-alkyl; $(C_1-C_4)$-Alkoxycarbonyl-$(C_1-C_{10})$-alkyl; $(C_1-C_8)$-Alkylsulfonyl; $(C_1-C_8)$-Alkylsulfinyl; Hydroxy-$(C_1-C_{10})$-alkanoyl; $(C_1-C_8)$-Alkanoyloxy-$(C_1-C_{10})$-alkyl; $(C_1-C_{11})$-Alkanoyl; gegebenenfalls geschütztes Amino-$(C_1-C_{11})$-alkanoyl; Di-$(C_1-C_7)$-alkylamino-$(C_2-C_{11})$-alkanoyl; $(C_3-C_9)$-Cycloalkylcarbonyl; $(C_6-C_{10})$-Aryl-$(C_2-C_{11})$-alkanoyl; 2-Pyridyl-$(C_1-C_8)$-alkanoyl; 3-Pyridyl-$(C_1-C_8)$-alkanoyl; 4-Pyridyl-$(C_1-C_8)$-alkanoyl; gegebenenfalls durch Halogen, $(C_1-C_7)$-Alkyl, $(C_1-C_7)$-Alkoxy oder $(C_1-C_7)$-Alkoxycarbonyl substituiertes Benzoyl; Pyrrolyl-2-carbonyl; Pyridyl-3-carbonyl; Benzolsulfonyl; $(C_1-C_{10})$-Alkoxycarbonyl; substituiertes $(C_1-C_{10})$-Alkoxycarbonyl; $(C_6-C_{14})$-Aryl-$(C_1-C_6)$-alkoxycarbonyl steht; oder $R^1$ gemeinsam mit $R^{12}$ ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxydiert sein kann,
$R^2$ $(C_3-C_{12})$-Alkyl; mono-, bi- oder tricyclisches $(C_3-C_{18})$-Cycloalkyl, $(C_3-C_{18})$-Cycloalkylmethyl, $(C_3-C_{18})$-Cycloalkylethyl, wobei der Cycloalkylteil gegebenenfalls durch $(C_1-C_6)$-Alkyl substituiert ist; Dithiolanyl; $(C_6-C_{14})$-Arylmethyl; Dithiolanylmethyl; Dithiolanylethyl; Dithianyl; Dithianylmethyl oder Dithianylethyl ist,
$R^3$ und $R^4$ unabhängig voneinander Wasserstoff oder $(C_1-C_{12})$-Alkyl sind oder zusammen mit Bor, X und Y ein mono-, bi-oder tricyclisches, gesättigtes oder teilweise ungesättigtes, gegebenenfalls mono-, di-, tri- oder tetra-$(C_1-C_{12})$-alkyliertes Ringsystem mit 5-18 Ringgliedern bilden, das außer Bor, X, Y und Kohlenstoff noch ein -O- Glied, ein -$NR^{13}$-Glied oder ein -$CR^{14}R^{15}$-Glied enthalten kann,
X und Y unabhängig voneinander -O- oder -$NR^{13}$- sind,
$R^5$ und $R^9$ unabhängig voneinander Wasserstoff; $(C_1-C_{10})$-Alkyl, das gegebenenfalls ein- oder zweifach ungesättigt ist und das gegebenenfalls durch bis zu 3 gleiche oder verschiedene Reste aus der Reihe Hydroxy, $(C_1-C_7)$Alkoxy, $(C_1-C_7)$-Alkanoyloxy, Carboxy, $(C_1-C_7)$Alkoxycarbonyl, Cl, Br, Amino, Amidino, Guanidino, Carbamoyl, $(C_1-C_5)$-Alkoxycarbonylamino, $(C_7-C_{15})$-Aralkoxycarbonylamino und 9-Fluorenylmethoxycarbonylamino substituiert ist; $(C_3-C_{12})$-Cycloalkyl-$(C_1-C_3)$-alkyl, mono- oder bicyclisches $(C_6-C_{14})$-Aryl-$(C_1-C_3)$-Alkyl, das gegebenenfalls durch einen oder zwei gleiche oder verschiedene Reste aus der Reihe F, Cl, Br, I, Hydroxy, $(C_1-C_7)$-Alkoxy, $(C_1-C_7)$-Alkyl, $(C_1-C_7)$-Alkoxycarbonyl, Amino und Trifluormethyl substituiert ist, bedeuten; oder für $(C_1-C_3)$-Alkyl, substituiert mit dem Rest eines 5- oder 6-gliedrigen monocyclischen oder 9- oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie in Anspruch 1 für $(C_6-C_{14})$-Aryl beschrieben mono- oder disubstituiert ist, stehen,
$R^6$ Wasserstoff, Methyl oder Ethyl ist oder zusammen mit $R^5$ Pyrrolidin oder Piperidin, die jeweils noch mit Cyclopentyl, Cyclohexyl oder Phenyl anneliert sein können, bildet,
$R^7$ und $R^8$ unabhängig voneinander Wasserstoff, Hydroxy, Amino, Fluor, Hydroxymethyl oder Aminomethyl sind,
$R^{10}$ und $R^{11}$ unabhängig voneinander Wasserstoff, Hydroxy, Phenyl, 2- oder 3-Thienyl, 2-, 3- oder 4-Pyridyl, 1-, 2- oder 4-Imidazolyl, 1- oder 2-Naphthyl; 2- oder 3-Benzo[b]thienyl sind,
n und m unabhängig voneinander 0, 1, 2, 3 und 4 bedeuten können,
$R^{12}$ wie in Anspruch 1 definiert ist,
$R^{13}$ Wasserstoff oder $(C_1-C_{12})$-Alkyl ist,
$R^{14}$ und $R^{15}$ wie in Anspruch 1 definiert sind,
$A^2$ abwesend ist, wobei gleichzeitig $A^1$ nicht für einen Rest der Formel II steht, oder einen Rest der Formel VIII bedeutet, wobei $R^5$ und $R^6$ wie vorstehend beschrieben definiert sind,
sowie deren physiologisch verträgliche Salze.

3. Verfahren gemäß einem oder mehreren der Ansprüche 1 bis 2, dadurch gekennzeichnet, daß man eine Verbindung der Formel I herstellt, in welcher
$A^1$ wie in Anspruch 1 definiert ist,
$R^1$ $(C_1-C_8)$-Alkylsulfonyl; $(C_1-C_8)$-Alkylsulfinyl; Hydroxy-$(C_1-C_{10})$-alkanoyl; $(C_1-C_8)$-Alkanoyloxy-

$(C_1-C_{10})$-alkyl; $(C_1-C_{11})$-Alkanoyl; Amino-$(C_1-C_{11})$-alkanoyl; Di-$(C_1-C_7)$-alkylamino-$(C_2-C_{11})$-alkanoyl; $(C_3-C_9)$-Cycloalkylcarbonyl; $(C_6-C_{10})$-Aryl-$(C_2-C_{11})$-alkanoyl; 2-Pyridyl-$(C_1-C_8)$-alkanoyl; 3-Pyridyl-$(C_1-C_8)$-alkanoyl; 4-Pyridyl-$(C_1-C_8)$-alkanoyl; gegebenenfalls durch Halogen, $(C_1-C_7)$-Alkyl, $(C_1-C_7)$-Alkoxy oder $(C_1-C_7)$-Alkoxycarbonyl substituiertes Benzoyl; Pyrrolyl-2-carbonyl; Pyridyl-3-carbonyl; Benzolsulfonyl; $(C_1-C_{10})$-Alkoxycarbonyl; substituiertes $(C_1-C_{10})$-Alkoxycarbonyl; $(C_6-C_{14})$-Aryl-$(C_1-C_6)$-alkoxycarbonyl bedeutet,

$R^2$ $(C_3-C_{12})$-Alkyl; mono-, bi- oder tricyclisches $(C_3-C_{18})$-Cycloalkyl oder $(C_3-C_{18})$-Cycloalkylmethyl, wobei der Cycloalkylteil gegebenenfalls durch $(C_1-C_4)$-Alkyl substituiert ist; $(C_6-C_{14})$-Arylmethyl; Dithiolanyl; Dithiolanylmethyl; Dithianyl und Dithianylmethyl bedeutet,

$R^3$ und $R^4$ Wasserstoff sind oder gemeinsam mit Bor, X und Y ein mono-, bi- oder tricyclisches mono-, di-, tri- oder tetra-$(C_1-C_{12})$-alkyliertes, gesättigtes oder teilweise ungesättigtes Ringsystem mit 5-18 Ringgliedern bilden, das außer Bor, X, Y und Kohlenstoff noch ein -O- Glied, ein -$NR^{13}$-Glied oder ein -$CR^{14}R^{15}$-Glied enthalten kann,

X und Y für -O- stehen,

$R^5$ und $R^9$ unabhängig voneinander Wasserstoff, Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto,1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)ethyl, 3-Thienyl, 3-Thienylmethyl, 2-(3Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methyl-imidazol-4-yl)methyl, (3-Methyl-Imidazol-4-yl)methyl, Phenyl, 1-Naphthylmethyl, 2-Naphthylmethyl, 2-Phenylmethyl, 2-Thiazolylmethyl, 4-Thiazolylmethyl, 3-Pyrazolylmethyl, 4-Pyrimidinylmethyl, Indol-2-yl-methyl, 2-Benzo[b]thienylmethyl, 3-Benzo[b]thienylmethyl, 2-Furylmethyl sind.

$R^6$ Wasserstoff oder Methyl bedeuten oder gemeinsam mit $R^5$ und der diese Reste tragenden -N-CH-Gruppe ein Tetrahydroisochinolin- oder Azabicyclooctan-Gerüst bildet,

einer der Reste $R^7$ oder $R^8$ Wasserstoff bedeutet, der jeweils andere Rest Hydroxy, Amino, Fluor, Hydroxymethyl oder Aminomethyl,

$R^{10}$ und $R^{11}$ unabhängig voneinander Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl sind,

n und m unabhängig voneinander 0, 1 oder 2 bedeuten,

$R^{12}$ Wasserstoff oder Methyl ist, oder gemeinsam mit $R^1$ ein mono- oder bicyclisches gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann,

$R^{13}$ Wasserstoff oder $(C_1-C_4)$-Alkyl ist,

$R^{14}$ und $R^{15}$ wie in Anspruch 1 definiert sind,

$A^2$ abwesend ist, wobei gleichzeitig $A^1$ nicht für einen Rest der Formel II steht, oder einen Rest der Formel VIII bedeutet, wobei $R^5$ und $R^6$ wie in Anspruch 1 definiert sind,

sowie deren physiologisch verträgliche Salze.

4. Verfahren gemäß einem oder mehreren der Ansprüche 1 bis 3, dadurch gekennzeichnet, daß man eine Verbindung der Formel I herstellt, in welcher

$A^1$ für einen Rest der Formel II, III oder VI steht, worin

$R^1$ $(C_1-C_6)$-Alkylsulfonyl; $(C_1-C_6)$-Alkylsulfinyl; Amino-$(C_1-C_6)$-alkanoyl; $(C_1-C_6)$-Alkoxycarbonyl; $(C_6-C_{10})$-Aryl-$(C_1-C_6)$-alkoxycarbonyl bedeutet,

$R^2$ $(C_5-C_8)$-Cycloalkyl; $(C_5-C_{11})$-Cycloalkylmethyl; [$(C_1-C_4)$-Alkyl-cyclohexyl]methyl; $(C_3-C_4)$-Alkyl, das durch $(C_1-C_3)$-Alkyl substituiert ist; $(C_6-C_{10})$-Arylmethyl oder dithiolan-2-yl-methyl bedeutet,

$R^3$ und $R^4$ für Wasserstoff stehen oder zusammen mit Bor, X und Y einen 1,3,2-Dioxaborolan-Rest, der gegebenenfalls durch 1 bis 4 Methylgruppen substituiert ist, einen [4,5-Diisopropyl]-1,3,2-dioxaborolan-, einen [(N-B)-(2,2′-imino-diethanolato)-boryl)]-, einen Pinandioxyboryl- oder einen 1,3,2-Dioxaborinan-Rest, dessen C-Atom in Position 5 durch $R^{14}$ und $R^{15}$ substituiert ist, bilden,

X und Y für -O- stehen,

$R^5$ Wasserstoff, n-Propyl, Isopropyl, n-Butyl, Isobutyl, sec.-Butyl, Carbamoylmethyl, 2-Carbamoylethyl, Benzyl, 2-Methylbenzyl, 3-Methylbenzyl, 2-Thienylmethyl, 3-Thienylmethyl, 1-Naphthylmethyl, 2-Naphthylmethyl, Imidazol-4-yl-methyl oder 2-Thiazolylmethyl bedeutet,

$R^6$ für Wasserstoff oder Methyl steht,

$R^{10}$ und $R^{11}$ unabhängig voneinander 1-Naphthyl oder 2-Thienyl bedeuten,

n und m für 1 stehen,

$R^{12}$ Wasserstoff bedeutet,

$R^{14}$ und $R^{15}$ unabhängig voneinander Wasserstoff, $(C_1-C_8)$-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)amino oder Bis(2-hydroxyethyl)amino bedeuten,

und

$A^2$ für einen Rest der Formel VIII steht, wobei $R^5$ und $R^6$ wie in Anspruch 3 definiert sind,

sowie deren physiologisch verträgliche Salze.

5. Verfahren zur Herstellung eines pharmazeutischen Mittels, enthaltend eine Verbindung der Formel I gemäß einem oder mehreren der Ansprüche 1 bis 4, dadurch gekennzeichnet, daß man diese und einen Träger in eine geeignete Darreichungsform bringt.